# EUROPEAN PATENT SPECIFICATION

(11) **EP 1 506 227 B1**
(45) Date of publication and mention of the grant of the patent: **02.08.2006**
(21) Application number: 03730422.7
(22) Date of filing: 14.05.2003
(51) Int. Cl.: C07K 14/44, C12Q 1/00

(54) **METHODS FOR IDENTIFYING AND DEVELOPING COMPOUNDS THAT INTERACT WITH VOLTAGE-GATED POTASSIUM CHANNELS OF THE KV4 FAMILY**
METHODEN FÜR DIE IDENTIFIZIERUNG UND DIE ENTWICKLUNG VON KOMPONENTEN DIE MIT POTENTIAL ABHÄNGIGEN KALIUM KANALEN DER KV4 FAMILIE INTERAGIEREN
METHODES D'IDENTIFICATION ET DE MISE AU POINT DE COMPOSES INTERAGISSANT AVEC LES CANAUX POTASSIUM POTENTIEL-DEPENDANTS DE LA FAMILLE KV4

(30) Priority: 15.05.2002 GB 0211123; 15.05.2002 US 378131 P; 15.05.2002 US 378076 P
(43) Date of publication of application: 16.02.2005
(73) Proprietor: Devgen NV, 9052 Gent-Zwijnaarde (BE)
(72) Inventor: KALETTA, Titus, Jan, B-9820 Merelebeke (BE); DEWULF, Nathalie, Els, B-8450 Bredene (BE); PLAETINCK, Geert, Karel, Maria, B-9820 Bottelare (BE); BOGAERT, Thierry, Andre, Olivier, Eddy, B-8500 Kortrijk (BE)
(74) Representative: White, Nina Louise
(86) International application number: PCT/IB2003/002453
(87) International publication number: WO 2003/097682

(56) References cited:
- WO-A-00/73493
- US-B1- 6 368 823
- WEI A ET AL: "Eight potassium channel families revealed by the C. elegans genome project" , NEUROPHARMACOLOGY, PERGAMON PRESS, OXFORD, GB, VOL. 35, NR. 7, PAGE(S) 805-829 XP002095808 ISSN: 0028-3908 abstract page 811, column 2, paragraph 3 page 817, column 1, paragraph 2 page 821, column 1, paragraph 3 page 823, column 1, paragraph 2 page 825, column 1, paragraph 3 -column 2, paragraph 1 table 1 figure 6
- WEI A D ET AL: "FUNCTIONAL PROPERTIES AND TISSUE DISTRIBUTION OF C. ELEGANS POTASSIUM CHANNEL HOMOLOGS OF HUMAN KVLQT1" , BIOPHYSICAL JOURNAL, NEW YORK, US, US, VOL. 74, NR. 2, PAGE(S) A206 XP000905423 ISSN: 0006-3495 the whole document
- BIANCHI A ET AL: "A Potassium Channel-MiRP Complex Controls Neurosensory Function in Caenorhabditis elegans" , THE JOURNAL OF BIOLOGICAL CHEMISTRY, VOL. 278, NR. 14, PAGE(S) 12415-12424, 4 APRIL 2003 XP002254280 abstract page 12415, column 2, paragraphs 2,4 page 12416, column 1, paragraph 5 -page 12418, column 2, paragraph 3 page 12421, column 2, paragraph 1 -page 12422, column 1, paragraph 1 page 12423, column 2, paragraph 1 figures 2-4

## Description

The present invention relates to methods for identifying and developing compounds that are useful in the pharmacological and/or veterinary fields, to assays and screens for use in such methods, and to compounds that may be identified using such methods.

In particular, the invention relates to methods of identifying compounds that may "interact with" (as further defined below) voltage-gated potassium channels of the *Shal* (Kv4) family (further referred to below as *"Kv4 channel(s)").* Such compounds may be useful in the prevention and/or treatment of disease states or disorders that are associated with such channels, some of which will be further mentioned below.

The invention also relates to methods for determining whether a compound can interact with a Kv4 channel, for instance to determine whether said compound is an agonist or an antagonist (e.g. a blocker or an opener) of/for a Kv4 channel.

In some other non-limiting aspects, the invention relates to assays and screens that embody these methods and/or that can be used in performing these methods; and to transgenic nematodes that are suitable for use in these methods, such as transgenic nematodes of the species *Caenorhabditis elegans* ("*C. elegans*"),

In one particularly advantageous embodiment, the invention provides methods, assays and/or screens that are suitable for - e.g. that are or can be configured for - (automated) screening of sets or libraries of compounds at medium to high throughput (as further defined below). Such methods, assays and/or screens - as well as the compounds identified therewith - may for instance be used in the discovery, development and/or preparation of (compositions containing) active compounds for pharmacological, veterinary and/or agrochemical use, as further described below.

The invention therefore also relates to the compounds identified using the methods, assays and/or screens of the invention, and to the use of such compounds in the development and/or the preparation of compositions for pharmaceutical and/or veterinary use.

Further aspects, embodiments, applications and advantages of the invention will become clear from the further description hereinbelow.

Kv4 channels, as well as their (encoding) sequences, their biological function/activity and their disease associations have been described in the art, see for example Bahring et al., *J.Biol.Chem.,* Vol. 276, no. 26, 233888-23894 (2001); Baldwin et al., *Neuron* 7: 471-483 (1991); Dixon et al., *Circ. Res.* 79: 659-688 (1996); Dilks et al., *J. Neurophysiol.* 81: 1974-1977 (1999); Kuo et al., *Cell,* Vol. 107, 801-813 (2001); Pak et al., *Proc. Natl. Acad Sci USA* 88; 4386-4390 (1991); Ohya et al., *FEBS Lett. 420:47-53 (1997)*; Roberts and Tamkun, *Proc. Natl. Acad. Sci USA* 88; 1798-1802; Rudy et al.; *Mol. Cell. Neurosci.* 2; 89-102 (1991); Serodio et al., J. Neurophysiol 75: 2174-2179 (1996); Serodio and Rudy, *J. Neurophysiol.* 79: 1081-1091 (1998); and Takimoto et al., *Circ. Res.* 81: 553-539 (1997), and the further references cited therein.

Generally, being voltage-gated potassium channels, Kv4 channels are *inter alia* involved in membrane depolarisation and repolarisation events, e.g. as part of and/or following neuronal firing and/or as part of the cycle of muscle contraction/relaxation.

In particular, and as mentioned in the above references, Kv4 channels are believed to be involved in the native A-type currents that are generated by various types of primary cells (Dilks et al., supra), in particular in muscle and neuronal cells. Kv4.2 and Kv4.3 transcripts have been found in most neurons, and in particular in CNS neurons (see Serodio and Rudy, supra, who discuss the distribution of Kv4 channels in rat brain); as well as in heart muscle (see Dixon et al, and by Serodio et al., both supra, who discuss the abundance and distribution of Kv4 transcripts in the hearts of rat, dog and human). It has also been found that, compared to Kv-type channels from other families such as Kv1-type channels, Kv4 channels activate and inactivate at subthreshold potentials, inactivate with time constants that change very little as a function of voltage (even at very negative potentials), and recover very fast from inactivation (see Rudy and Serodio, supra).

In neuronal cells, and in particular in neurons in the brain, Kv4 channels are *inter alia* believed to play an important role in the modulation of the firing rate, action potential initiation, shaping burst pattern and postsynaptic signal integration (Dilks et al., and Bahring et al., supra), and are believed to be associated with the physiological states/disorders that result from such activity (Serodio and Rudy, supra).

In the heart, the Kv4 channels are *inter alia* believed to play a major role in the calcium-independent A-type currents in the cardiac muscle (the "transient outward current" or "Iₜₒ"), and in particular in the cardiac ventricular muscle, and are thus believed to be involved in early repolarization and hence the overall duration of the action potential and the length of the refractory period (Serodio and Rudy, supra). Because of this, Kv4 channels are believed to be associated with (the susceptibility to) cardiac disorders such as arrythmia and other types of heart failure (Kuo et al., supra).

So far, three mammalian Kv4 genes - referred to as Kv4.1 (also known as mSha1), Kv4.2 (also known as RK5) and Kv4.3, respectively - have been cloned and characterized, i.e. from rat and dog (Dixon et al, Serodio et al., Ohya et al. and Takimoto et al., all supra) and from human (Dilks et al., and Bahring et al., supra; see also for example WO 98/42833 and US-A-6,395,477).

The sequences of (genes encoding) mammalian Kv4 channels are also available from publicly accessible databases such as GenBank/NCBI, e.g. Kv4.1 from mouse (accession number NP_032449 and A3 8372); Kv4.1 from human (accession number BAA96454, AAF65617 and AF65516); Kv4.2 from mouse (accession number NP_062671 and AAD16972), Kv4.2 from rat (accession number NP_113918); Kv4.2 from human (accession number AAD22053 and CAB56841); Kv4.3 from mouse (accession numbers NM_019931 and AF384170), Kv4.3 from rat (accession number U42975) and Kv4.3 from human (accession number XM_052127).

The above references also indicate that further channels from the Kv4 family may be identified and cloned in future, for example from neurons in the brain that show Kv4-like subthreshold-operating A channels, but do not show abundant expression of Kv4.1, Kv4.2 and/or Kv4.3 transcripts (see Serodio and Rudy, supra) or other suitable tissues/cells.

As mentioned above, the Kv4 channels in mammals also have a high degree of sequence identity (>70%) with, and thus are considered closely related to, the *Shal-*like gene product, which encodes a potassium channel in *Drosophila melanogaster* (see Baldwin et al, supra, and also WO 01/58952).

Also, the present inventors have sequenced a *C. elegans* gene having orthology with mammalian Kv4 genes, referred to herein as KV4.x. The sequence of the gene as sequenced is given in SEQ ID no. 3. This sequence differs somewhat from the sequences predicted on the basis of the AceDB and EMBL sequence databases (vide AceDB no. Y73B6BL.19; EMBL no. AC084197).

In view of the biological functions and disease associations mentioned above, it is expected that compounds that act as agonists and antagonists of Kv4 channels (and/or of the biological function(s) and/or pathways associated with these channels), and in particular compounds that can (fully or partially) "block" and/or "open" Kv4 channels, can be useful as pharmaceutically active agents, in particular for the prevention and/or treatment of cardiac disorders such as arrythmia, hypertension-induced heart disorders such as hypertension-induced cardiac hypertrophy (e.g. ventricular hypertrophy), and disorders of the nervous system such as epilepsy, stroke, traumatic brain injury, anxiety, insomnia, Alzheimer's disease and Parkinson's syndrome.

Nevertheless, the prior art does not describe any methods by which such compounds could be (effectively) identified and/or developed. In particular, the art does not describe any methods for screening libraries of chemical compounds for agonists and/or antagonists of Kv4 channels at medium to high throughput (in this respect, it should be noted that the throughput of conventional techniques for screening ion channels, such as "patch clamp" or FLIPR-techniques, is rather limited).

It is therefore a general object of the invention to provide methods that allow for the identification and/or the development of compounds that can interact with a Kv4 channel, and/or that make it possible to determine, in a qualitative and/or quantitative manner, whether a (given) compound interacts or can interact with a Kv4 channel.

According to the invention in its broadest sense, it has been found that this object can be achieved by the use - in such a method, assay or screen - of a nematode that has been transformed with at least one nucleotide sequence encoding a Kv4 channel, e.g. by contacting said nematode with the compound(s) to be tested while said nematode expresses said Kv4 channel (i.e. in at least one of its cells, parts, tissues or organs), and then determining (any changes in) at least one detectable/observable (biological) property or characteristic of said nematode, i.e. as a result of the exposure of the nematode to the compound(s) to be tested, i.e. compared to a suitable reference (such as wild-type or any other suitable strain, including but not limited to the strain used to express the Kv4 channel).

Thus, in a first aspect, the invention relates to a nematode worm , characterized in that said worm expresses a heterologous nucleotide sequence that encodes a functional voltage-gated potassium channel of the Kv4 family which is Kv4.2 or Kv4.3 or a mutant thereof having a degree of sequence identity, at the amino acid level, of at least 50% with the corresponding human Kv4.2 or Kv4.3 channel.

Said nematode worm is preferably from the genus *Caenorhabditis,* and is more preferably (a strain of) *Caenorhabditis elegans.* As such, said nematode may be wild-type *C. elegans* (and/or may have been derived therefrom), a suitable mutant line or strain (and/or may have been derived therefrom), and/or may be a suitable transgenic line or strain (and/or may have been derived therefrom), all as further described below.

Preferably, (the nucleotide sequence encoding) the Kv4 channel is heterologous to the nematode used, as further defined below. As such, (the nucleotide sequence encoding) the Kv4 channel may be.derived from any suitable biological source, as further described below. Preferably, (the nucleotide sequence encoding) the Kv4 channel is derived from a mammal, more preferably from a human.

Also, instead of (a nucleotide sequence encoding) a naturally occurring Kv4 channel, a (natural or synthetic) Kv4 mutant thereof may be used, as further defined below.

The voltage gated potassium channel of the Kv4 family is a preferably a Kv4.2 or Kv4.3 channel, with a Kv4.3 channel being particularly preferred. The nucleotide sequence encoding human Kv4.3 used in the Experimental Part below is shown in SEQ ID no. 4. The amino acid sequence of human Kv4.3 is shown in SEQ ID no. 5.

The Kv4 channel should be expressed in at least one cell, part, tissue or organ of the nematode, again as further described below. For this purpose, the nucleotide sequence encoding the Kv4 channel may be expressed in the worm under the control of one or more suitable regulatory elements, such as a suitable promoter operable in the nematode, also as further described below.

Preferably, the expression of the Kv4 channel leads to at least one detectable change in/compared to the nematode used to express said channel. Preferably, the detectable change caused by the expression of the Kv4 channel (referred to below as *"detectable phenotypical change")* is a detectable biological change, and in particular a detectable phenotypical, physiological, behavioural and/or biochemical change, as further described below.

More preferably, said at least one detectable phenotypical change is associated with - e.g. will be a result of - the biological activity of the expression of the Kv4 channel in the nematode (i.e. in at least one cell, part, tissue or organ thereof). Some preferred, but non-limiting examples of such detectable phenotypical changes will be described hereinbelow.

In a further aspect, the invention also relates to a method for determining whether a compound or compounds can interact with a voltage gated potasssium channel of the Kv4 family, which method comprises the steps of:
(a) contacting at least one nematode worm as described herein with said (at least one) compound or compounds; and
(b) detecting and/or observing at least one detectable change in or of said nematode worm.

The invention also relates to a method for screening a set of compounds for the presence in said set of one or more compounds that can interact with a voltage gated potasssium channel of the Kv4 family, said method comprising the steps of
(a) contacting one or more of the compounds in said set of compounds with at least one nematode worm as described herein, and
(b) detecting and/or observing whether any of the compounds in said set causes a detectable change in or of said nematode worm.

The invention also relates to a method for identifying a compound that can interact with a voltage gated potasssium channel of the Kv4 family, said method comprising the steps of
(a) contacting with at least one nematode worm as described herein with said compound, and
(b) detecting and/or observing whether said compound causes a detectable change in or of said nematode worm;
wherein said detectable change identifies a compound that can interact with said Kv channel.

The invention also relates to a method for identifying a compound that can be used in the prevention and/or treatment of a disease or disorder that is associated with and/or that is caused by (a defect in) a Kv4 channel, said method comprising the steps of:
(a) contacting at least one nematode worm as described herein with said compound, and
(b) detecting and/or observing whether said compound causes a detectable change in or of said nematode worm;
wherein said detectable change identifies a compound that can be used in such prevention and/or treatment (and/or that can be used in the preparation of a pharmaceutical composition for such prevention and/or treatment).

The disease or disorder is preferably a disease or disorder that is associated with and/or that is caused by (a defect in) Kv4.1, Kv4.2 and in particular Kv4.3, and is more in particular chosen from the diseases and disorders mentioned hereinabove and/or in the prior art referred to hereinabove.

In particular, the disease or disorder may be a cardiac disorder that is associated with and/or that is caused by (a defect in) a Kv4 channel, such as arrythmia, and/or a disease or disorder of the nervous system (particularly including but not limited to the central nervous system) that is associated with and/or that is caused by (a defect in) a Kv4 channel, including but not limited to the diseases mentioned hereinabove and/or in the prior art referred to hereinabove.

Preferably, the detectable change caused by the compound(s) to be tested (referred to below as *"significant biological change"*) is a detectable biological change, and in particular a detectable phenotypical, physiological, behavioural and/or biochemical change, as further described below.

More preferably, said at least one significant biological change is representative for - e.g. will be a result of - the interaction of the compound(s) with the Kv4 channel expressed in the nematode used. Some preferred, but non-limiting examples thereof will be mentioned below.

In yet another aspect, the invention relates to the use of a transgenic nematode as described herein in the above methods.

In a further aspect, the invention relates to a compound that is identified using the transgenic nematode and/or using the methods described herein. These compounds may be agonists and/or antagonists (e.g. blockers or openers) of the pertinent Kv4 channel, and may for example be used in (the preparation of a pharmaceutical preparation for) the prevention and/or treatment of diseases and disorders associated with (defects in) Kv4 channels, including but not limited to the diseases and disorders mentioned herein and/or in the prior art referred to hereinabove.

Further non-limiting aspects, embodiments, applications and advantages of the invention will become clear from the following detailed description of the invention.

In the invention, any suitable nematode worm may be used. Preferably, the nematode worm is a microscopic nematode, i.e. a nematode with a size (in adult state) of no more than 3 mm, preferably no more than 2 mm, and usually between 0.1 and 1.5 mm. Suitable examples thereof will be clear to the skilled person.

More preferably, the nematode used is of the genus *Caenorhabditis,* and is more in particular *Caenorhabditis elegans.* In this embodiment, the invention may provide one or more of the advantages associated with the use of *C*. *elegans,* such as:
- *C. elegans* has a short life-cycle of about 3 to 4 days.
   This not only means that these nematodes (and suitable mutants, transgenics and/or stable lines thereof) can be cultivated/generated quickly and in high numbers, but also allows assays using *C. elegans* to test, in a relatively short period of time and at high throughput, the nematode worms over one or more, and up to all, stages of life/development, and even over one or more generations. Also, because of this short life span, in *C. elegans* based-assays, compounds may be tested over one or more, and up to essentially all, stages of development, without any problems associated with compound stability and/or (bio)availability. Also, due to its small size, *C. elegans* may be cultured and handled in the wells of a multi-well plate, allowing for such assays to be performed in an automated fashion;
- *C. elegans* is transparant, allowing -with advantage- for visual or non-visual inspection of internal organs and internal processes, and also the use of markers such as fluorescent reporter proteins, even while the worms are still alive. Also, as further mentioned below, such inspection may be carried out in automated fashion using suitable equipment such as plate readers;
- Techniques for handling, cultivating, maintaining and storing (e.g. as frozen samples, which offers great practical advantages) *C. elegans* are well established in the art, for instance from the standard *C. elegans* handbooks referred to below. For example, *C. elegans* may be used as a one or more samples with essentially fully isogenic genotype(s);
- *C. elegans* is a well-established and well-characterized model organism. For example, the genome of *C*. *elegans* has been fully sequenced (with 70% of human gene pathways being conserved), and also the complete lineage and cell interactions (for example of synapses) are known. In addition, *C. elegans* has full diploid genetics, and is capable of both sexual reproduction (e.g. for crossing) as well as reproduction as a self-fertilizing hermaphrodite. Furthermore, various techniques for genetic modification of *C. elegans,* including transformation and selection techniques, are readily available from the art. All this may provide many advantages, not only for the use of *C. elegans* in genetic and/or biological studies, but also for the use of *C*. *elegans* in the discovery, development and/or pharmacology of (candidate) drugs for human or animal use.
- *C. elegans* can easily take up the compounds to be tested from the surrounding medium, for example via the gastrointestinal tract (e.g. by pharynx pumping followed by active or passive transport through the wall of the gastrointestinal tract) and/or by diffusion through the cuticle. In this way, it has also been shown that drugs that are active in humans also show a biological effect on *C. elegans* (i.e. on the biologically relevant phenotype);
or any combination thereof.

For the sake of convenience, the invention will now be further described below with reference to *C. elegans.* However, in view of the above, it should be clear that the invention in its broadest sense is not limited to the use of this specific nematode.

For general information on *C. elegans* and techniques for handling this nematode worm, reference is made to the standard *C. elegans* handbooks, such as W.B. Wood et al., *"The nematode Caenorhabditis elegans",* Cold Spring Harbor Laboratory Press (1988); D.L. Riddle et al., "C. *ELEGANS II",* Cold Spring Harbor Laboratory Press (1997); *"Caenorhabditis elegans, Modern Biological analysis of an organism":* ed. by H. Epstein and D. Shakes, Methods in Cell Biology, Vol 48, 1995; and and *"C. elegans, a practical approach",* ed. by I.A. Hope, Oxford University Press Inc. New York, USA, 1999.

In addition, some further techniques and methodology for performing *in vivo* assays using *C. elegans* are generally described in the following Applications by applicant: PCT/EP99/09710 (published on 15 June 2000 as WO 00/34438); PCT/EP99/04718 (published on January 15, 2000 as WO/00/01846); PCT/IB00/00575 (published on October 26, 2000 as WO 00/63427); PCT/IB00/00557 (published on October 26,2000 as WO 00/63425); PCT/IB00/00558 (published on October 26,2000 as WO 00/63426); as well as in for instance PCT/US98/10080 (published on 19-11-1998 as WO 98/51351), PCT/US99/13650, PCT/US99/01361 (published on 29-07-1999 as WO99/37770), and PCT/EP00/05102.

In the invention, any suitable *C. elegans* strain or line may be transformed with one or more nucleotide sequences encoding a Kv4 channel, and/or may otherwise be brought to express a Kv4 channel, so as to provide a nematode worm suitable for use in the invention. Suitable lines or strains will be clear to the skilled person, and may include, but are not limited to, wild-type and N2 strains, as well as mutant and/or transformed lines or strains of *C. elegans,* including but not limited to the mutant lines or strains listed in the standard *C. elegans* handbooks referred to above.

Some preferred *C. elegans* lines or strains for use in the invention include, but are not limited to:
- wild type and commonly used laboratory strains such as N2 and Hawaii;
- constitutive pumping strains, and in particular unc-31 and HD8 (LMBP 5447CB, also called *bg46* or *hdr(bg46),* see PCT/IB00/00557 by Applicant). Of these strains/mutants, any suitable allele can be used such as *unc31*(e928).
- *unc* mutants, in particular *unc-19, unc-36* and *unc-119.* Of these mutants, any suitable allele can be used, for example *unc36*(e251) or *unc*-*119*(ed3).
- *egl*-mutants, and in particular *egl-16, egl-19, egl-36* and *egl*-119. Of these mutants, any suitable allele can be used, for example *egl-19*(n582), egl-36(sa577), *egl-36*(n728) or *egl19*(n2368).
- *eat*-mutants, and in particular *eat-16.* Of these mutants, any suitable allele can be used, for example *eat16*(sy438).
- *exp*-mutants, in particular *exp-2* mutants, and more in particular *exp-2* loss-of-function mutants. Of these mutants, any suitable allele can be used, for example *exp-*2(sa26ad1426) or *exp-2*(sa26ad1201) (for the latter, see Davis, Dent and Avery, Worm Breeder's Gazette 14(4): 72 (1996).
- *pha*-mutants, and in particular *pha-*1. Of these mutants, any suitable allele can be used, for example *pha-1* (e2123).

Thus, two preferred, but non-limiting classes of mutants are the "muscle-related" mutants (such as exp-2, unc-19 and other muscle related mutants mentioned above), and "neuron-related" mutants.

One preferred, but non-limiting, group of mutants for expression of the Kv4 channel in accordance with the invention includes those in which one or more of the native ion channels involved in neuronal function, and in particular one or more of the native ion channels involved in the polarisation and/or repolarisation of neuronal and/or muscle membranes (including but not limited to orthologs of Kv4 channels in the nematode, such as Kv4.x and other native ion channels), have been altered (for example downregulated or fully knocked out) compared to wild type or N2. For example, these may be mutants in which one or more of the potassium channels involved in membrane repolarization are fully or partially closed (either in a transient manner, such as by RNAi, or in a constitutive manner, such as by a suitable mutation). In these latter mutants - of which the *exp-2* mutant above is a preferred, but non-limiting example - expression of the Kv4 channel may partially or full restore or "rescue" the mutant phenotype. Generally, such mutants may be identified by means of electropharyngyogram ("EPG") and/or intracellular electrical recordings ("IER") of pharyngeal muscle, in that they will have an EPG and/or IER that is different from the EPG and/or IER of wildtype or N2.

For "electropharyngeograms" ("EPG") and "intracellular electrical recordings" ("IER") reference is made to Raizen and Avery, Neuron, Vol.12, 483-495 (1994) and to the standard *C. elegans* handbooks referred to above, and in particular to the chapter by Avery and Thomas in Riddle et al., *C. ELEGANS II* (Chapter 24, pages 679-716); and to Hope et al., in particular pages 171-176. Alternatively, neuronal or muscle action may be determined using well-known patch-clamp techniques.

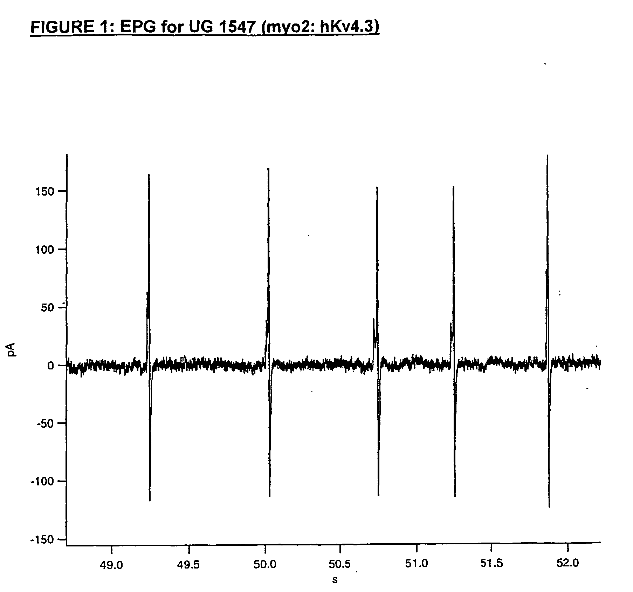

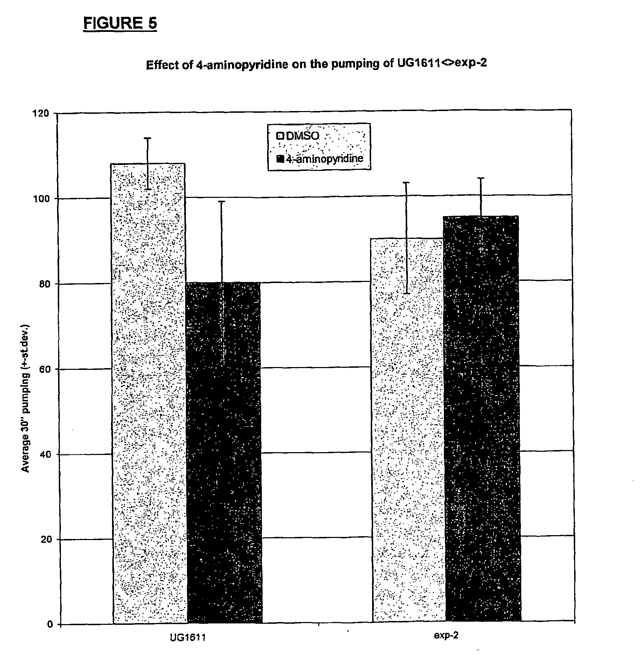

For example, on EPG, mutants like *exp-2* will have a "repolarization" (or "relaxation") peak (peak "R", vide Avery and Thomas, figure 5 on page 688) that is reduced or even essentially absent compared to wild-type of N2 and/or will have a "refractory" (or "relaxation") period (i.e. the time between the exitation peak "E" and the relaxation peak R, also sometimes referred to - both hereinbelow and in the prior art - as the "plateau phase", the "P-phase" or the "duration") that is changed compared to wild-type of N2. Reference is also made to Davis, Dent and Avery, Worm Breeder's Gazette 14(4):72 (October 1, 1996), which reference shows comparative EPG's from wild-type and exp-2 mutants, as well as Figure 1, that shows an EPG of a N2 background expressing a human Kv4.3 channel under the control of a myo-2 promoter (invention).

Also, in one preferred, but non-limiting embodiment of the invention, the strains used for expression of the Kv4 channels will show reduced pharynx pumping and/or reduced drinking (vide below), and preferably both, compared to wild type/N2. Because of its defects in membrane repolarization, the *exp-2* mutant is also a non-limiting example thereof.

In yet another embodiment, the strains used for expression of the Kv4 channels show a decreased sensitivity and/or activity of (one or more of the) the sensory neurons. A preferred, but non-limiting example of such a "sensory neurons-related" mutant strain is *osm-3.*

The above and other mutants (and suitable alleles thereof) suitable for use in the invention will generally be described in the art - for example in the *C. elegans* handbooks referred to above - and may for example be obtained from the Caenorhabditis Genetics Center ("CGC"), St. Paul, Minnesota, USA.

The (nucleotide sequence encoding the) Kv4 channel that is used to transform the nematode may be any (nucleotide sequence encoding a) voltage-gated potassium channel of the Kv4 family. As such, it may be (a nucleotide sequence encoding) a Kv4.1, Kv4.2 or Kv4.3 channel, or any further voltage-gated potassium channel of the Kv4 channel that is described in the art or that may be identified after the date of filing of the present application.

Preferably, the Kv4 channel is Kv4.2 or Kv4.3, with Kv4.3 being particularly preferred.

The (nucleotide sequence encoding the) Kv4 channel may be obtained from any suitable biological source, which will usually be a multicellular animal, and in particular a multicellular animal that contains one or more neuronal cells and/or muscle cells.

Preferably, (the nucleotide sequence encoding) the Kv4 channel is derived from a vertebrate animal, and in particular from a warm-blooded animal, and more in particular from a mammal, such as a rat, a mouse, a rabbit, a sheep, a pig, a goat, a dog, a non-human primate or a human (which is particularly preferred). However, the invention in its broadest sense is not limited thereto, and also includes - for example - the use of Kv4 channels from:
- other vertebrate animals, including but not limited to birds, fish such as Zebrafish, reptiles and amphibians such as Xenopus;
- invertebrate animals, including but not limited to insects such *Drosophila, Heliothis,* housefly, mosquito, or species of aphid (e.g. Sha-1 like channels), nematodes such *C. elegans* (e.g. Kv4.x channels) and harmful and/or parasitic nematodes such as *Ostertagia, Haemonchus* and *Trichostringylus.*

It is also within the scope of the invention to transform the nematode with:
- (one or more nucleotide sequences encoding) one or more parts or fragments of a naturally occurring Kv4 channel; and/or with
- (one or more nucleotide sequences encoding) a natural or synthetic mutant, variant, analog, homolog or ortholog of a naturally occurring Kv4 channel, and/or with one or more parts or fragments thereof;
all of which are collectively referred to herein as *"mutant Kv4 channel(s)".* These mutant Kv4 channels may - for instance - differ from a naturally occurring Kv4 channel by addition, substitution, insertion and/or deletion of one or more amino acid residues (or, in case of a nucleotide sequence, of one or more bases/nucleotides) at one or more positions.

Preferably, any such mutant Kv4 channel is derived from a naturally occurring mammalian Kv4 channel, and in particular from a naturally occurring human Kv4 channel. For example, natural orthologs of human Kv4 channels from other species may be identified using bio-informatic techniques and/or by the use of probes/primers designed on the basis of the human Kv4 sequence.

Also, preferably, any (nucleotide sequence(s) encoding a) mutant Kv4 channel used in the invention will have a degree of sequence identity, at the amino acid level, of at least 50%, preferably at least 60%, even more preferably at least 70%, with even more preference at least 80%, most preferably at least 90%, and in particular more than 95%, with the sequence of the corresponding human Kv4 channel (e.g. Kv4.1, Kv4.2 and preferably Kv4.3) (or, alternatively, to the Kv4.x sequence of SEQ ID No. 3). For this purpose, the percentage of *"sequence identity"* between the amino acid sequence of a mutant Kv4 channel and the amino acid sequence of the human Kv4 channel may be calculated by dividing [*the number of amino acid residues in the sequence of the mutant Kv4 channel that are identical to the amino acid residue at the corresponding amino acid position of the amino acid sequence of the human Kv4 channel*] by [*the total number of amino acid residues in the amino acid sequence of the human Kv4 channel]* and multiplying by [*100*%], in which each deletion, insertion, substitution or addition of an amino acid residue in the mutant sequence - i.e. compared to the given sequence - is considered as a difference at a amino acid position. Alternatively, the degree of sequence identity may be calculated using a known computer algorithm for sequence alignment such as NCBI Blast v2.0, using standard settings.

For these purposes, amino acid sequence of human Kv4.3 is shown in SEQ ID no. 5. For the sequences of other human Kv4 channels, reference is made to the prior art referred to above.

Naturally occurring mutant Kv4 channels for use in the invention may be identified and isolated from a suitable biological source in a manner known per se, for example by direct expression from a DNA library, by screening a DNA library with a suitable probe, or isolation of suitable mRNA followed by cDNA synthesis using reverse transcriptase. Synthetic mutant Kv4 channels may for example be obtained - e.g. based on and/or starting from the sequence of a naturally occurring Kv4 channel - by techniques such as automated DNA synthesis, site-directed mutagenesis, combining two or more parts of one or more naturally occurring Kv4 channels (e.g. to provide a "hybrid Kv4 channel"), introduction of mutations that lead to the expression of a truncated expression product, or the introduction of mutations by means of a PCR reaction using one or more "mismatched" primers. These and other techniques will be clear to the skilled person; reference is for instance made to the standard handbooks, such as Sambrook et al, "Molecular Cloning: A Laboratory Manual" (2nd.ed.), Vols. 1-3, Cold Spring Harbor Laboratory Press (1989) and F. Ausubel et al, eds., "Current protocols in molecular biology", Green Publishing and Wiley Interscience, New York (1987).

Furthermore, as already indicated above, it is envisaged that further channels belonging to the Kv4 family may be identified and/or (further) characterized after the date of filing of the present application (e.g. from mammals and in particular from humans), for example as a result of functional genomics programs and/or target discovery programs. It is expected that the invention may equally be applied - i.e. in a manner analogous to the manner described herein - to such new Kv4 channels, e.g. to identify and develop compounds that may interact with such channels and/or to determine whether a compound can interact with such channels.

Preferably, (the nucleotide sequence encoding) the Kv4 channel is "heterologous" to the nematode used, by which is meant that (the nucleotide sequence encoding) said Kv4 channel does not naturally/natively occur in said nematode (and in which the term "heterologous" is also meant to include: (a) synthetic Kv4 channels as described herein; and (b) mutants, variants, analogs, homologs, orthologs, parts and/or fragments of the Kv4 channels that are native to the nematode used). However, in its broadest sense, the invention is not limited thereto and also includes the use of homologous Kv4 channels (i.e. that naturally occur in the nematode used); for this purpose, such a native Kv4 channel- e.g. "Kv4.x" referred to herein; vide SEQ ID no. 3 - may for example be brought to overexpression (e.g. compared to the native strain used), for example under the regulation of a promoter with which said channel is not natively associated.

Furthermore, although it is generally preferred in the invention to transform the nematode with a single Kv4 channel, it is also within the scope of the invention to transform the nematode with two or more different Kv4 channels of interest, and/or with one or more Kv4 channels of interest and one or more further heterologous genes, including but not limited to one or more components of the pathway(s) in which said Kv4 channel is (suspected to be) involved, and/or one or more auxiliary proteins, acceptor molecules and/or subunits of Kv4 channels, including but not limited to KChiP's such as KChiP1, KChiP2, amd KChiP3, as well as Kvβ (see for example Bahring et al, supra; and Kuo et al., supra, and An et al., Nature, Vol. 403, p. 553-556, (2000)). The (nucleotide sequence(s) encoding the) one or more further heterologous genes may be again be naturally occuring (e.g. from the same biological source as the Kv4 channel and/or from another suitable biological source) and/or may be synthetic sequences, including mutants, parts, fragments etc. as described above.

Generally, the nucleotide sequence(s) encoding the Kv4 channel(s) may be genomic sequences (which may contain native introns) and/or cDNA sequences, with cDNA sequences being preferred. Such nucleotide sequences may also - for example - contain artificial introns, may contain donor/acceptor splice sites to maximize changes of heterologous transgenic expression, and/or may have adapted codon usage in order to promote expression in the nematode.

It will also be clear to the skilled person that during/upon expression of the Kv4-encoding sequence(s) in the nematode - and depending upon the specific Kv4-encoding sequence(s) and the specific nematode used - events such as gene splicing, SL1/SL2 splicing, and/or post-translational modifications, as well as intercellular transport (in particular for localization on or at the cell surface/cell membrane) may occur. All such events are included within the present invention, provided they result in a *"functional Kv4 channel",* by which is generally meant herein a Kv4 channel that - upon expression as described herein - can be tested for interaction with the compound(s) of interest, i.e. as part of the method(s) or assay(s) of the invention.

The (one or more genes encoding the) Kv4 channel may be provided for use in the invention as a single nucleic acid (sequence), which is generally preferred, or as two or more separate nucleic acids.

Generally, Kv4 channel will be expressed in the nematode by transforming said nematode (or at least one cell, tissue, organ or part thereof, including but not limited to the gonads and/or oocytes) with one or more nucleic acids that encode the desired Kv4 channel, upon which the transformed nematode (or a descendent thereof) may be exposed to, or maintained under, conditions such that the expression of said Kv4 channel is obtained, i.e. in at least one part, tissue, organ or cell of said nematode.

Preferably, such nucleic acids encoding the Kv4 channel are in the form of a genetic construct, which may be DNA or RNA, and are preferably double-stranded DNA. Such a construct may also be in a form suitable for transformation of the nematode used, in a form suitable for integration into the genomic DNA of the nematode used, in a form suitable for independent replication, maintenance and/or inheritance in the nematode used (e.g as part of the extrachromosomal array), and/or in a form suitable independent replication, maintenance and/or inheritance in another desired host organism (such as a micro-organism used for cloning, for example *E. coli).*

For instance, said genetic construct may be in the form of a plasmid, vector, transposon, and/or linear fragments, such as linear PCR-fragments.

The genetic construct(s) used in the invention may further contain- i.e. beside the one or more nucleic acid sequence(s) encoding the Kv4 channel - one or more further suitable elements of genetic constructs known per se, including but not limited to suitable regulatory elements (such as a suitable promoter, enhancer, terminator, etc.; which regulatory elements may also be used to regulate the expression of the Kv4 channel in the nematode), 3'- or 5'-UTR sequences, leader sequences, selection markers, expression markers/reporter genes, splice/donor sites, and/or elements that may facilitate or increase (the rate of) transformation or integration.

These and other suitable elements for such genetic constructs will be clear to the skilled person, and may for instance depend upon the type of construct used, the nematode to be transformed; the cell(s), tissue(s), part(s) or organ(s) in which the Kv4 channel are to be expressed; the manner in which the Kv4 channel is to be expressed (e.g. via constitutive, transient or inducible expression); and/or the transformation technique used. Some specific, but non-limiting examples of such elements for use in nematodes, and in particular in *C. elegans,* will be mentioned below. Other such suitable elements can for example be found in the standard handbooks referred to above (e.g. Sambrook et al. and Ausubel et al), in the standard *C. elegans* handbooks referred to above (e.g. Wood et al., Riddle et al., Epstein and Shakes, and Hope), in the applications by Applicant referred to above, as well as in other patent literature (see for example WO 95/07463, WO 96/23810, WO 95/07463, WO 95/21191, WO 97/11094, WO 97/42320, WO 98/06737 and/or WO 98/21355).

Preferably, in the genetic constructs of the invention, the one or more optional elements mentioned above are *"operably linked"* to the nucleotide sequence(s) encoding the Kv4 channel and/or to each other, by which is generally meant that they are in a functional relationship with each other. For instance, a promoter is considered *"operably linked"* to a coding sequence if said promoter is able to initiate or otherwise control/regulate the transcription and/or the expression of a coding sequence (in which said coding sequence should be understood as being "under the control of" said promotor). Generally, when two nucleotide sequences are operably linked, they will be in the same orientation and usually also in the same reading frame. They will usually also be essentially contiguous, although this may also not be required.

Preferably, the optional further elements of the genetic construct(s) used in the invention are such that they are capable of providing their intended biological function in the nematode. For instance, a promoter, enhancer and/or terminator should be *"operable"* in the nematode, by which is meant that - in at least one cell, tissue, organ or part of the nematode - said promoter (for example) should be capable of initiating or otherwise controlling/regulating the transcription and/or the expression of a nucleotide sequence - e.g. a coding sequence - to which it is operably linked (as defined above).

Such a promoter may be a constitutive promoter or an inducible promoter, and may be homologous (which is usually preferred) or heterologous to the nematode used. Some non-limiting examples of suitable promoters and include, but are not limited to:
- inducible, specific promoters such as: CYP35A2, CYP35A3 mtl-1, mtl-2
- constitutive, specific promoters such as: ceh-28, dpy-7, egl-15, ges-1, glh-2, glp-1, mec-7, myo-2, myo-2 min., myo-3, osm-6, pes-10 min., pIRES, serca, unc-119, unc-129 full, unc-129-BstEII frag, unc-18, unc-183, unc-4, unc-27, unc-49, unc-54 enhancer/promotor, unc-71, unc-8, xol-1
- inducible, non-specific (i.e. "ubiquitous") promoters such as: hsp-16.2, hsp-16.41, hsp-16.48, the Tet-On/Tet-Off^{™} promoter system
- constitutive, non-specific promoters such as: let-858
- transient promoters such as vit-2;

Other suitable heterologous promoters for use in *C*. *elegans* can be found in the standard *C. elegans* handbooks and in the applications by Applicant referenced above.

Preferred promoters for use in the invention are promoters that direct the expression of the Kv4 channel (at least) towards:
- muscle tissue (for example, the sca-1 promoter), and in particular to the pharynx muscle (for example, the myo-2 promoter); the body wall muscle (for example, the myo-3 promoter); and/or the vulva muscle (for example, the egl-15 or egl-36 promoter);
- neurones (for example, the unc-18 or unc-119 promoters), and in particular:
   (a) the M4 neurones, which *inter alia* control the isthmus (for example, the ceh-28 promoter) and/or
   (b) the sensory neurones (for example ASI specific promoters such as the daf-7, gpa-4 or str-3 promoters; AWA specific promoters such as odr-7, odr-3, odr-4, odr-10, osm-9, gpa-5 or gpa-6 promoters; AWB specific promoters such as str-1, gcy-10, odr-1 or tax-2; or other suitable odr, gpa, osm, str, or tax promoters);
- the gut (for example, the vit-2 promoter);

Another promoter that may be used in the invention, in particular for the expression in the neuronal cells and/or muscle cells, is the promoter of the native *C. elegans* Kv4.x sequence referred to herein (or an operable fragment thereof).

Yet another class of suitable promoters are the Troponin promoters, including but not limited to the Troponin C, Troponin I and/or Troponin T promoters, including but not limited to TnI-1, TnI-2, TnI-4, TnC or pat-10, TnT-1 or TnT-2, which may be used for constitutive expression in muscle tissue (such as pharynx muscle, body wall muscle, vulva muscle, anal muscle), either throughout the lifecycle or during specific stages thereof (e.g. embyro and larvae for TnI-1, and larvae and adult for TnI-2).

A selection marker should be capable of distinguishing - e.g. allow the detection and/or selection of - nematodes that contain the genetic construct. For instance, such a selection marker may be any gene that can be used to select - under suitable conditions such as the use of a suitable selection medium, or a suitable temperature - nematodes that contain - e.g. as the result of (a successful) transformation, inheritance or crossing - the genetic construct containing the marker.

For example, some preferred, but non-limiting examples of selection markers suitable for use in/selection of *C*. *elegans* include *pha-1.* Other suitable selection markers for use in *C. elegans* will be clear to the skilled person; reference is for instance made to the standard *C*. *elegans* handbooks and to the applications by Applicant referred to above.

A leader sequence should be such that - in the nematode or in the pertinent cell(s), tissue(s), organ(s) or part(s) thereof - it may allow for the desired post-translational modifications; it may direct mRNA and/or the translated amino acid sequence to a desired part or organelle of a cell; and/or it may allow for secretion of the expression product from said cell. As such, it may for instance be any pro-, pre-, or pre/pro-sequence operable in said cell or organism.

For example, some preferred, but non-limiting examples of leader sequences suitable for use in *C. elegans* include the daf-2 leader sequence, the leader sequence of the KV4.x gene referred to herein and/or the *C. elegans* splice leader ("SL") sequences. Other suitable leader sequences for use in *C. elegans* will be clear to the skilled person; reference is for instance made to the standard *C. elegans* handbooks and to the applications by Applicant referred to above. In this respect, it will be clear that for the expression of a Kv4 channel, a leader sequence that allows for any necessary post-translational modifications and/or that directs the expressed Kv4 channel to the cell membrane, although it may not be required, will often be particularly preferred.

An expression marker or reporter gene should be such that - in the nematode or in the pertinent cell(s), tissue(s), organ(s) or part(s) thereof - it allows for the expression of (a gene or nucleotide sequence present on) the genetic construct to be detected, and should optionally also allow for the localisation of the expression product, e.g. in (a) specific cell(s), tissue(s), organ(s) or part(s) of the nematode, or in specific parts or organelles of such cells (such as - in case of the present Kv4 channels - in or on the cell membrane). Such a reporter gene may also be expressed as a protein fusion with (at least part of) the Kv4 channel.

For example, some preferred, but non-limiting examples of reporter genes/expression markers suitable for use in *C. elegans* include the fluorescent proteins well known in the art, such as the naturally occuring and/or commercially available "GFPs". Other suitable reporter genes for use in *C*. *elegans* will be clear to the skilled person; reference is for instance made to the standard *C. elegans* handbooks and to the applications by Applicant referred to above.

Suitable examples of other elements of genetic constructs that may be used in the constructs of the invention - such as terminators, transcriptional and/or translational enhancers and/or integration factors - will also be clear to the skilled person and may include, but are not limited to, terminators such as a polyA sequence or a 3'-UTR (for example derived from *C. elegans,* such as the *unc-54* 3'-UTR), enhancers such as UL6, and integration factors such as the *C. elegans* SL sites and *C. elegans* outrons; reference is again made to the standard *C. elegans* handbooks and to the applications by Applicant referred to above.

Also, in the invention, the nucleotide sequence encoding the Kv4 channel may be operably linked to at least one other sequence that encodes a further amino acid sequence such as a protein or polypeptide, so as to provide - upon expression - a protein fusion of said Kv4 channel said further amino acid sequence. For example, said further sequence may have/provide a specific and/or desired biological activity, or may be a reporter sequence such as a GFP.

The constructs of the invention can be provided in a manner known per se, which will generally involve techniques such as restricting and linking nucleic acids/nucleic acid sequences, as will be clear to the skilled person. Reference is again made to the standard handbooks, such as Sambrook et al. and Ausubel et al. mentioned above. The sequences encoding the further elements present in the constructs of the invention may be isolated from a suitable biological source, from a vector or plasmid described in the art, or may be synthesized using well known nucleic acid synthesis techniques.

The nematode may be transformed with the (genetic construct containing) the nucleic acid(s) encoding the heterologous Kv4 channel in any suitable manner, which may depend upon the nematode used and on some of the other considerations referred to above for the genetic constructs used. Some preferred, but non-limiting techniques for the transformation of *C*. *elegans* include microinjection, irradiation (e.g. with gamma-radiation), ballistic transformation, soaking, feeding, electroporation, or any other suitable transformation technique known per se, with microinjection and ballistic transformation (e.g. to generate integrated lines) being particularly preferred. Such techniques, and in particular microinjection, may also include micro-injection into the oocyte or gonad of a (parent) worm, thus introducing the nucleic acid(s) into the offspring. For a further description, reference is again made to the standard *C. elegans* handbooks referred to above.

Also, the transformation of *C. elegans* may involve the use of specific mutant strains or lines which are particularly suited/adapted for transformation, for integration and/or for the specific transformation/integration technique used. A preferred, but non-limiting, examples thereof is *unc-119,* which is particularly suited for ballistic transformation, and of which any suitable allele can be used, such as *unc-119*(ed3)*.*

Upon transformation, the nucleic acid(s) encoding the Kv4 channel may be integrated into the genome of the nematode (preferably in a stable manner, e.g. so as to provide an integrated line, which may sometimes be preferred in practice) or may be maintained and/or inherited essentially independently (again preferably in a stable manner), for example as part of the extrachromosomal array.

Another technique for generating a nematode that expresses the Kv4 channel may be crossing a first nematode that contains (the nucleic acid(s) encoding) the Kv4 channel (for example integrated in the genome) with another nematode of the same species, to as to provide offspring that at least contains the nucleic acid sequence(s) encoding the Kv4 channel, and that most preferably is also capable of expressing said Kv4 channel under suitable conditions, as described hereinbelow. Suitable techniques for crossing and subsequently selecting of *C*. *elegans -* i.e. to provide descendants having one or more properties of interest, such as expression of the Kv4 channel - will be clear to the skilled person. General reference is again made to the standard *C. elegans* handbooks referred to above.

Accordingly, in the method of the invention, not only a nematode directly obtained as a result of transformation may be used, but also further generations, progeny and/or offspring thereof (and these should be considered included within the term "nematode" as used herein). In one preferred, but non-limiting embodiment, these further generations, progeny and/or offspring are part of and/or constitute a stable line or strain, i.e. a line or strain (capable of) expressing the Kv4 channel.

To obtain expression of the heterologous Kv4 channel, the nematode may generally be cultured, kept and/or maintained under conditions such that expression of the Kv4 channel is obtained. Generally, such conditions will depend upon the nematode used and upon the regulatory elements that control the expression of the Kv4 channel, and may include the presence of a suitable source of food or nutrients (although sometimes, it may be advantageous to deprive the organism during a certain period of time of one or more such nutrients or food components) and/or other factors required for the growth or maintenance of the nematodes, a suitable medium, a suitable temperature, and the presence of a suitable inducing factor or compound (i.e. if the nucleotide sequence encoding the Kv4 channel is under the control of an inducible promoter). Generally, such conditions will be clear to the skilled person; reference is again made to the standard *C. elegans* handbooks and to the applications by Applicant referred to above.

For example, suitable conditions may include the use of a suitable liquid, solid, semi-solid or viscous medium such as agar plates, M9, S-buffer or a viscous medium with viscosity greater than M9 (e.g. as described in PCT/IB00/00575 and other applications by Applicant, and as measured at the cultivation and/or incubation temperature used, e.g. using an Ostwald, Ubbelohde or Brookfield viscosimeter) on a plate, in a petri-dish, in a tube, in a flask, in the well of a multi-well plate, or in another suitable vessel or container; a temperature of between 15 and 30 °C (usually about 20-25 °C), and the presence of a suitable source of food such as bacteria - for example *E. coli -* in an amount of between 0.05 and 0.5 % w/v, preferably about 0.125 % w/v.

Under such conditions, the Kv4 channel may be expressed in a constitutive manner (which is usually preferred), in a transient manner (for instance only during a specific phase of the lifecycle of the nematode) and/or only when suitably induced, dependant on the promoter used. In any case, the Kv4 channel should be present and functional (as referred to above) in at least one cell, tissue, organ or part of the nematode at the time the nematode is used in the method(s)/assay(s) of the invention, i.e. at the time the nematode is contacted with the compound(s) to be tested.

As already indicated above, under such suitable conditions, the Kv4 channel may be expressed in the entire nematode, or only in one or more specific part(s), organ(s), tissue(s) or cell(s) thereof. Preferably, in the invention, the Kv4 channel is (at least) expressed in:
- at least one muscle cell or muscle tissue, and preferably in (at least one cell of) the pharynx muscle, the body wall muscle and/or the vulva muscle
- at least one neuron/neuronal cell, and preferably in (at least one of) the M4 neurons; and/or
- at least (one cell of) the gastrointestinal tract (i.e. the wall thereof), such as (the wall of) the gut.

Although the invention in its broadest sense is not limited thereto, expression of the Kv4 channel in the nematode as described herein preferably leads to a *"detectable phenotypical change"* (as referred to above) in the nematode, i.e. compared to either wild-type/N2 and/or compared to the original "background" (i.e. strain or line) used to express the Kv4 channel; and as determined by suitable detection technique, some non-limiting examples of which are given below. Generally, when said detectable phenotypical change is measured using such a suitable detection technique, said change will be a change of at least 1%, preferably at least 5%, and may be up to 10% or more, of the value(s) measured, compared to the corresponding value(s) measured for wild-type/N2 and/or the original background.

Said detectable phenotypical change is preferably a detectable biological change, and more in particular at least one phenotypical, physiological, behavioural and/or biochemical change in the nematode, for example as generally referred to in the applications by Applicant mentioned above.

More preferably, said at least one detectable phenotypical change is associated with - and will usually be a result of - the biological activity the Kv4 channel in the nematode, e.g. on the cell(s), part(s), tissue(s) or organ(s) in which said Kv4 channel is expressed.

For example, when the Kv4 channel is expressed in the muscle and/or in the neurons, said at least one detectable phenotypical change is preferably a change that is associated with the function of at least one muscle or muscle tissue and/or with the function of at least one neuron or type of neurons in the worm. When the Kv4 channel is expressed in the gut, said at least one detectable phenotypical is preferably a change that is associated with the function of the digestive tract of the nematode.

In a preferred embodiment, said detectable phenotypical change is a detectable change in one or more of movement, pharynx pumping (e.g. expressed in number of pharynx contractions or "pumps" per unit time), drinking (e.g. as measured by an assay measuring uptake of a fluorescent dye as described in PCT/IB00/00575 by Applicant), egg laying, egg hatching, defecation, mating behaviour, starvation, lethality. Some other non-limiting biological changes may involve the response to external stimuli, such as mechanical and/or chemical stimuli (e.g. chemotaxis); the response to presence of food; the response of (changes in) temperature; the response to exposure to certain chemical substances; the entry into and/or the escape from the dauer stage; changes in growth; changes in life cycle; changes in metabolism; changes in neuronal function; and/or changes muscle function (e.g. contraction and relaxation) as may be seen in EPG and/or IER. Techniques for detecting and measuring such detectable phenotypical change(s) will be clear to the skilled person; reference is again made to the standard *C. elegans* handbooks referred to above. Also, the applications by Applicant referenced above generally describe automated/high throughput techniques for measuring the preferred detectable phenotypical changes mentioned above.

More preferably, the detectable phenotypical change that results from the expression of the Kv4 channel is a change in neuronal function and/or muscle function, including but not limited to a change in membrane polarization and/or repolarization (e.g. as seen on EPG and/or IER); neuronal firing; muscle contraction and/or relaxation; pharynx pumping; drinking; movement; defecation and/or egglaying, with a change in drinking, movement and/or egglaying being especially preferred, as these may be conveniently determined in an automated, medium-to-high throughput fashion.

Also, in one preferred aspect, the detectable phenotypical change may a change in membrane repolarization events, as may for instance be seen on EPG and/or IER, in particular an increase in the repolarization peak (R) as seen on EPG and/or a change, and in particular a decrease, in the time between the exitation peak (E) and the repolarization peak (R), compared to the background used for expressing the Kv4 channel. For example, as already mentioned above, the EPG's of *exp-2* like mutants show a repolarization peak that is reduced or even essentially absent compared to wild-type of N2. Expression of a Kv4 channel in an *exp-2* background may lead to a partial or full restoration of said peak (vide Figures 1A to 1C).

In another preferred, but non-limiting embodiment of the invention, expression of the Kv4 channel increases the rate of pharynx pumping, in particular when a (mutant) background that has reduced pharynx pumping compared to wild-type/N2. For example, in this non-limiting embodiment, expression of the Kv4 channel may increase the rate of pharynx pumping compared to the (mutant) background, but to a rate that is still lower than the pharynx pumping rate for wild type/N2.

In another preferred, but non-limiting embodiment of the invention, expression of the Kv4 channel increases drinking (for example as measured by the uptake of a fluorescent label such as Calcein AM), in particular for a when a (mutant) background that has reduced drinking compared to wild-type/N2. For example, in this non-limiting embodiment, expression of the Kv4 channel may increase drinking compared to the (mutant) background, but to a level that is still lower than the level of drinking of wild type/N2.

The following Table 1 gives a list of some preferred, but non-limiting combinations of (1) cells/tissues in which (the nucleotide sequence encoding) the Kv4 channels may be expressed; (2) promoters for use in such expression; (3) *C. elegans* strains that may be used a starting line or strain ("background") for such expression; and (4) the detectable phenotypical change that may be the result of the expression of the Kv4 channel in said cells/tissue.

The following human Kv4.3 expressing *C. elegans* lines/strains have been deposited with the Belgian Coordinated Collections of Microorganisms ("BCCM") LMBP-Collection, Universiteit Gent, K. L. Ledeganckstraat 35, B-9000, Gent, in accordance with the provisions of the Budapest Treaty on the International Recognition of the Deposit of Microorganisms:
- strain UG1598, deposited on April 25, 2002, under accession number LMBP 5851CB, which is a pha-1(e2123ts)III stain transformed with a human Kv4.3 channel under the control of a myo-2 promoter;
- strain UG1611, deposited on April 25, 2002, under accession number LMBP 5852CB, which is a exp-2(sa26ad1426)V stain transformed with a human Kv4.3 channel under the control of a myo-2 promoter.

**TABLE 1**

| **Expression in:** | **Promoter:** | **Background:** | **Phenotypical change:** |
|---|---|---|---|
| pharynx muscle | myo-2, TnI-4, TnT-3 | wild-type, N2, pha-1, HD8, | muscle function, in particular pharynx function (e.g. as seen on EPG and/or IER), pharynx pumping, drinking, starvation and/or lethality |
| pharynx muscle | myo-2, TnI-4, TnT-3 | *exp-2, exp2*;HD8 | muscle function, in particular pharynx function (as seen on EPG/IER), rescue of pumping defect |
| body wall muscle | myo-3, TnI-1, TnI-2 or unc-27, TnC or pat-10, TnT-1 or TnT-2 | wild-type, N2, pha-1 | muscle function, movement, hatching, lethality and/or egg laying |
| body wall muscle | myo-3, TnI-1, TnI-2 or unc-27, TnC or pat-10, TnT-1 or TnT-2 | exp-2, unc-19 | muscle function, EPG, movement, lethality, egg laying, defecation. |
| vulva muscle | egl-15, egl-36 | wild-type, N2, pha-*1, egl-16* | egg laying |
| vulva muscle | egl-15, egl-36 | *unc-36, egl-19* | rescue of egg laying defect |
| all muscles | sca-1, TnI, TnC, TnT | wild-type, N2, pha-1, HD-8, exp-2, unc-19 | muscle function (e.g. as seen on patch clamp or EPG), pharynx pumping, drinking, lethality, hatching starvation, egg laying, movement, defecation |
| M4 neurons | ceh-28 | wild-type, N2, pha-1 | neuronal function, pharynx pumping and/or drinking |
| gut | vit-2 | wild-type, N2, pha-1 | function of the digestive tract, defecation |
| sensory neurones | daf-7, gpa-4, gpa-5, gpa-6, str-1, str-3, odr-1, odr-3, odr-4, odr-7, odr-10, gcy-10, tax-2, osm-9 | wild-type, N2, pha-1, osm-3, sensitized dauer mutants. | dauer, chemotaxis, response to food, pharynx pumping, drinking |
| any neuronal cell | unc-119, unc-18 | wild-type, N2, pha-1 | neuronal function, pharynx pumping, drinking, starvation, lethality, movement, hatching, lethality and/or egg laying |

The transgenic nematodes expressing the Kv4 channel generated in accordance with the above may be used to identify compounds that "interact with" the Kv4 channel, and/or to determine whether a (given or pre-determined) compound "interacts with" the Kv4 channel.

In the context of the present disclosure, when a compound is said to *"interact with"* a protein of interest such as the Kv4 channel, it is generally meant that said compound in some way affects, changes, alters and/or otherwise influences at least one property, characteristic and/or activity of said protein, and in particular at least one biological property, characteristic and/or activity of said protein.

For this, the compound may for instance bind to, ligate with, form a complex with, form one or more hydrogen bonds with, and/or otherwise associate with the protein of interest, i.e. such that at least one (biological) property, characteristic and/or activity of said protein is affected, changed, altered or otherwise influenced. For example, the compound may bind to and/or associate with the protein of interest at or near the active site (e.g. in a manner comparable to protein-substrate interaction, for example so as to be competitive with the binding of the substrate), fully or partially block the protein at or near or at its active site, bind to some other (binding) site of the protein, influence the protein in an allosteric manner, act as a co-factor or inhibit the binding of a co-factor, promote or inhibit the association of the protein with other proteins, etc.; although the invention in its broadest sense is not particularly limited to the manner in which the compound interacts with/associates with the protein.

In particular, for an ion channel such as the pertinent Kv4 channels, said compound(s) may fully or partially block or open the channel, increase or decrease the amount of ions that (can) pass through the channel in a given period of time; and/or otherwise change the opening and/or closing, change the activity/activation and/or change the the sensitivity of the channel. Said influence may be competitive or non-competitive with the native substrate(s), may be transient or permanent, and/or may be reversible or irreversible (with transient and reversible usually being preferred).

Accordingly, the compound(s) may act as an agonist of the Kv4 channel, as an antagonist of the Kv4 channel and/or as a (reversible and/or irreversible) inhibitor of the Kv4 channel, although the invention in its broadest sense is not limited thereto. For instance, the compound potentiates, sensitizes or desensitizes the Kv4 channel, or otherwise upregulates or downregulates the activity and/or the sensitivity of the Kv4 channel.

Preferably, the above *"interaction"* of the compound(s) with the Kv4 channel is such that it can be measured or otherwise detected (i.e. qualitatively and/or quantitatively) by a detection suitable technique, for example using *Xenopus* oocytes and/or using EPG and/or IER in *C. elegans.*

More preferably - as in the invention, the interaction of the compound(s) with the protein is determined *in vivo* using the nematode *C. elegans -* said interaction is such that it (also) leads to a detectable change in the nematode(s) used. In particular, said detectable change (referred to herein as a *"significant biological change")* may be a detectable biological change in the nematode, e.g. at least one phenotypical, physiological, behavioural and/or biochemical change in the nematode.

Generally, when said significant biological change is measured using a suitable detection technique, said change will be a change of at least 1%, preferably at least 5%, and may be up to 10% or more, of the value(s) measured, compared to the corresponding value(s) measured for the strain used (but without exposure to the compound(s)).

Even more preferably, said at least one significant biological change in the nematode will be associated with - and will usually be a result of - the change(s) in the activity, sensitivity, etc., of the Kv4 channel that is caused by the compound(s).

For example, when the Kv4 channel is expressed in the muscle and/or in the neurons, said at least one significant biological change is preferably a change that is associated with the function of at least one muscle or muscle tissue and/or with the function of at least one neuron or type of neurons in the worm. When the Kv4 channel is expressed in the gut, said at least one significant biological change is preferably a change that is associated with the function of the digestive tract of the nematode.

In this respect, it should however be noted that generally, the *"significant biological change"* that is caused by the compound(s) to be tested may or may not be the same (e.g. essentially the same phenotypical, physiological, behavioural and/or biochemical property) as the *"detectable phenoypical change"* that is caused by the expression of the Kv4 channel in the nematode. For example, in one preferred embodiment, the "detectable phenotypical change" may be a defect in the nematode (such as a defect in pharynx pumping, movement or egglaying), and the significant biological change caused by compound may be to fully or partially "rescue" said defect.

In a preferred embodiment, the significant biological change is a detectable change in one or more of movement, pharynx pumping (e.g. expressed in number of pharynx contractions or "pumps" per unit time), drinking (e.g. as measured by an assay measuring uptake of a fluorescent dye as described in PCT/IB00/00575 by Applicant), egg laying, egg hatching, defecation, mating behaviour, starvation, lethality. Some other non-limiting biological changes may involve the response to external stimuli, such as mechanical and/or chemical stimuli (e.g. chemotaxis); the response to presence of food; the response of (changes in) temperature; the response to exposure to certain chemical substances; the entry into and/or the escape from the dauer stage; changes in growth; changes in life cycle; changes in metabolism; changes in neuronal function; and/or changes muscle function (e.g. contraction and relaxation) as may be seen in EPG and/or IER. Techniques for detecting and measuring such detectable change(s) have already been referred to above.

More preferably, the significant biological change is a change in neuronal and/or muscle function, including but not limited to a change in membrane polarization and/or repolarization (e.g. as seen on EPG and/or IER); neuronal firing; muscle contraction and/or relaxation; pharynx pumping; drinking; movement; defecation and/or egglaying, with a change in drinking, movement and/or egglaying being especially preferred.

Also, in one preferred aspect, the significant biological change may a change in membrane repolarization events, as may for instance be seen on EPG and/or IER, in particular an increase in the repolarization peak (R) as seen on EPG and/or a change, and in particular a decrease, in the time between the exitation peak (E) and the repolarization peak (R), compared to the background used for expressing the Kv4 channel. For example, as already mentioned above, the EPG's of *exp-2* like mutants show a repolarization peak that is reduced or even essentially absent compared to wild-type of N2. Expression of a Kv4 channel in an *exp-2* background may lead to a partial or full restoration of said peak (vide Figures 1A to 1C).

For example, the following Table 2 gives a list of some preferred, but non-limiting combinations of (1) cells/tissues in which (the nucleotide sequence encoding) the Kv4 channels may be expressed; (2) promoters used for such expression; (3) *C*. *elegans* strains that used a starting line or strain ("background") for such expression; and (4) the relevant significant biological change that the compound(s) to be tested may cause in such a nematode.

**TABLE2**

| **Expression in:** | **Promoter:** | **Background:** | **Phenotypical change:** |
|---|---|---|---|
| pharynx muscle | myo-2, TnI-4, TnT-3 | wild-type, HD8, | muscle function, in particular pharynx function (e.g. as seen on EPG and/or IER), pharynx pumping, drinking, starvation and/or lethality |
| pharynx muscle | myo-2, TnI-4, TnT-3 | *exp-2, exp2*;HD8 | muscle function, in particular pharynx function (as seen on EPG/IER), rescue of pumping defect |
| body wall muscle | myo-3, TnI-1, TnI-2 or unc-27, TnC or pat-10, TnT-1 or TnT-2 | wild-type | muscle function, movement, hatching, lethality and/or egg laying |
| body wall muscle | myo-3, TnI-1, TnI-2 or unc-27, TnC or pat-10, TnT-1 or TnT-2 | exp-2, unc-19 | muscle function, EPG, movement, lethality, egg laying, defecation. |
| vulva muscle | egl-15, egl-36 | wild-type, *egl-16* | egg laying |
| vulva muscle | egl-15, egl-36 | *unc-36, egl-19* | rescue of egg laying defect |
| all muscles | sca-1, TnI, TnC, TnT | wild-type, HD-8, exp-2, unc-19 | muscle function (e.g. as seen on patch clamp or EPG), pharynx pumping, drinking, lethality, hatching starvation, egg laying, movement, defecation |
| M4 neurons | ceh-28 | wild-type | neuronal function, pharynx pumping and/or drinking |
| gut | vit-2 | wild-type | function of the digestive tract, defecation |
| sensory neurones | daf-7, gpa-4, gpa-5, gpa-6, str-1, str-3, odr-1, odr-3, odr-4, odr-7, odr-10, gcy-10, tax-2, osm-9 | wild-type, N2, pha-1, osm-3, sensitized dauer mutants. | dauer, chemotaxis, response to food, pharynx pumping, drinking |
| any neuronal cell | unc-119, unc-18 | wild-type | neuronal function, pharynx pumping, drinking, starvation, lethality, movement, hatching, lethality and/or egg laying |

Generally, the method for determining whether a compound or compounds can interacts with a Kv4 channel will comprise the steps of:
(a) contacting at least one nematode worm expressing a Kv4 channel with said (at least one) compound or compounds; and
(b) detecting and/or observing at least one significant biological change (as defined above) in or of said nematode worm.

The method for screening a set of compounds for the presence in said set of one or more compounds that can interact with a Kv4 channel will generally comprise the steps of:
(a) contacting one or more of the compounds in said set of compounds with at least one nematode worm as described herein, and
(b) detecting and/or observing whether any of the compounds in said set causes a significant biological change in or of said nematode worm.

As mentioned above, in step (b) both these methods, the significant biological change observed in said nematode will preferably at least be indicative of - and even more preferably be representative for - the interaction of the compound(s) with the Kv4 channel, in a qualitative and preferably (also) quantitative manner. The above steps (a) and (b) will now be described in more detail below.

In step a), the nematode (i.e. at least one cell, part, tissue or organ thereof) is contacted with the compound(s) to be tested. This may be carried out in any suitable manner, such as:
- by bringing the compound(s), optionally in the a suitable formulation such as a solution or suspension, (directly) into contact with the body and/or the cuticle ("skin") of the nematode, and/or by otherwise introducing the compound into the surroundings of the nematode, such as by incorporating the compound(s) into the medium in or on which the nematode is kept, grown, cultivated and/or maintained (which medium may also be such that it suitably induces expression of the Kv4 channel). It is well known that under these circumstances, nematodes can take up compounds directly from their surroundings through their cuticle (e.g. by diffusion or active transport, sometimes also referred to as "soaking") and/or via openings in their cuticle, such as the amphid sensory neurons;
- by injecting or by otherwise introducing the compound(s) into the body of the nematode, for example into the blood stream or into any part(s) of the gastrointestinal tract;
- by feeding the compound(s) to the nematode and/or by including the compound(s) into a suitable food source, food composition or another suitable substance or material that the worms may take up through feeding or pharynx pumping (e.g. microspheres) and then feeding said food source, etc. to the nematodes.

These and other techniques for exposing the nematode to the compound(s) to be tested, and suitable conditions for use therein, will be clear to the skilled person. More in general, the invention is not particularly limited as to the manner in which the nematode is contacted with the compound(s) to be tested, as long as the compound(s) is absorbed or otherwise taken up by the nematode, such that said compound can come into contact with the Kv4 channel(s) expressed by said nematode; and preferably such that - when said compound is capable of interacting with said Kv4 channel(s) as defined herein - the exposure of the nematode to said compound leads to a significant biological change (as described above).

For instance, the time that the nematode is exposed to/contacted the compound(s) to be tested will depend on factors such as the nematode used (including the specific strain used), the manner in which the nematode is contacted with said compound(s), the amount of compound(s) to which the nematode is exposed (i.e. both in absolute amount and/or in concentration), the significant biological change to be measured, and similar factors. The concentration and/or the amount of the compound(s) to which the nematode is exposed will usually depend on factors such as the nematode used, the manner in which the nematode is contacted with said compound, the significant biological change to be measured, the (intended) time of exposure, and similar factors.

Generally, the skilled person will be able to determine a suitable time, amount and concentration for exposure to the compound, optionally after some routine initial experimentation involving a limited amount of trial-and-error and/or by comparison with one or more reference compounds that are known to provide interact with Kv4 channels (vide below).

The number of nematodes that is contacted with the compound(s) to be tested (i.e. the "sample size") is not critical and may for instance depend on the size of the container/vessel used. Usually, the sample will comprise between 2 and 500, in preferably between 3 and 300, more preferably between 5 and 200, even more preferably between 10 and 100 nematodes. When the assay is carried out in multi-well plate format (as described below), each well will usually contain between 15 and 75 worms, such as between 20 and 50 worms. Although not preferred, it is not excluded that a sample may consist of a single worm. Also, although usually not preferred, it is also possible to use only a part, tissue, organ or cell of a nematode of the invention, such as (isolated) muscle tissue, an isolated pharynx, etc..

Although the invention in its broadest sense is not limited thereto, it is usually preferred that the nematodes in each sample will of consist of worms that - at least at the start of the assay - are essentially the same, i.e. in that they: essentially are of the same strain (e.g. expessing the Kv4 channel); essentially are of an isogenic genotype; essentially show the same, Kv4-related phenotype; essentially are "synchronised" (i.e. at essentially the same stage of development, which however may - and usually will - change during the course of the assay, and not for all worms in the sample at the same rate and/or in the same way); essentially have been grown/cultivated in the same way (e.g. in the same medium).

Further conditions for contacting the nematode with the compound(s) to be tested - such as the temperature, the medium and the equipment used, etc. - may be suitably chosen by the skilled person, again optionally after some routine initial experimentation involving a limited amount of trial-and-enror, and again based on the considerations referred to above. For example, these conditions may - but do not necessarily have to be - generally be the same as the conditions for cultivating/maintaining nematodes as described hereinabove (and thus may also be such that expression of the Kv4 channel is induced/obtained). However, these conditions may involve the use of temperatures, media, buffers and reagents that are specifically suited for testing/screening of compounds, e.g. as described in the applications by Applicant referred to above. Furthermore, and although usually not preferred, it is also within the scope of the invention to expose the nematode, i.e. prior to or concurrent with the exposure to the one or more compounds to be tested, to one more compounds that upregulate or downregulate at least one native process or activity of the nematode, such as a natively present ion-channel. However, at a minimum, the conditions used should be such that they are physiologically acceptable to the nematodes used, at least during the time it takes to carry out the methods of the invention.

For example, some suitable, non-limiting conditions may be as follows:
- a contact time of between 1 minute and 24 hours, in particular between 10 minutes and 12 hours, more in particular between 0.5 hours and 8 hours, such as between 1 and 6 hours, for example about 2, 3, 4 or 5 hours;
- a concentration/amount of the compound(s) of between 0.01 and 1000 mM (depending on the toxicity and solvability of the compound tested), in particular between 1 and 500 mM, more in particular between 5 and 300 mM, such as between 5 and 100 mM, e.g. about 10, 20, 30, 40, 50, 60, 70, 80 or 90 mM; in which the compound(s) may optionally be (pre-)mixed with a suitable food source such as *E.coli* (used in the amounts indicated above);
- a temperature of between 15 and 30 °C, in particular between 17 and 25 °C, such as about 18, 19, 20, 21, 22, 23 or 24 °C;
- the use of a suitable liquid, viscous or semi-viscous medium, such as agar, M9, S-buffer, and/or one of the media referred to above; on a plate, in a petri-dish, in a tube, in the well of a multi-well plate, or in another suitable vessel or container;
- a sample size of between 10 and 1000 nematodes, in particular between 25 and 200 nematodes, more in particular between 75 and 150 nematodes.

Other suitable conditions for testing/screening of compounds using nematode worms are described in the applications by Applicant referred to above. For example, when the significant biological change to be measured is movement, pharynx pumping egglaying or defecation, some particularly suited conditions for such assays may be found in the International Application PCT/IB00/00575 (WO00/63427).

Once the nematodes have been exposed to the compound(s) to be tested, the significant biological change caused by said exposure may be measured/detected in any suitable visual or non-visual manner, depending on the phenotypical, physiological, behavioural and/or biochemical property/properties to be determined. Suitable techniques will be clear to the skilled person; reference is made to the *C. elegans* handbooks and the applications by Applicant referred to above, as well as to the further description herein.

For example, changes in neuronal function may be determined by EPG and/or IER. Changes in the rate of pharynx pumping may be determined by visually counting the number of pharynx contractions ("pumps") over time, using a microscope and a stopwatch. Changes in drinking may be measured by determining the uptake of a fluorescent probe such as Calcein-AM of time, as described in International Application PCT/IB00/0057 (WO00/63427) by Applicant, which also describes techniques for measuring movement, egglaying and defecation.

The significant biological change(s) thus measured/determined will be indicative of, and preferably will be a measure for, the interaction of the compound with the Kv4 channel, and will thus allow for compound(s) that interact with the Kv4 channel to be identified, and/or to determine the interaction of a compound with the Kv4 channel.

As to the compound(s) that may be tested in the invention: in one preferred, but non-limiting embodiment, these will be "small molecules", by which is generally meant herein a molecular entity with a molecular weight of less than 1500, preferably less than 1000. This may for example be an organic, inorganic or organometallic molecule, which may also be in the form or a suitable salt, such as a water-soluble salt; and may also be a complexe, chelate and/or a similar molecular entities, as long as its (overall) molecular weight is within the range indicated above.

In a preferred embodiment, such a "small molecule" has been designed according, and/or meets the criteria of, at least one, preferably at least any two, more preferably at least any three, and up to all of the so-called Lipinski rules for drug likeness prediction (vide Lipinksi et al., Advanced Drug Delivery Reviews 23 (1997), pages 3-25). As is known in the art, small molecules which meet these criteria are particularly suited (as starting points) for the design and/or development of pharmaceuticals for human use, and may for instance be used as starting points for hits-to-leads chemistry, and/or as starting points for lead development (in which the methods of the invention may also be applied).

Also, for these purposes, the design of such small molecules (as well as the design of libraries consisting of such small molecules) will preferably also take into account the presence of pharmacophore points, for example according to the methods described by I. Muegge et al., J. Med. Chem. 44, 12 (2001), pages 1-6 and the documents cited herein.

The term "small peptide "generally covers (oligo)peptides that contain a total of between 2 and 35, such as for example between 3 and 25, amino acids (e.g. in one or more connected chains, and preferably a single chain). It will be clear that some of these small peptides will also be included in the term small molecule as used herein, depending on their molecular weight.

In one preferred, but non-limiting embodiment, the invention is used to screen a set or library of (related or otherwise unrelated) small molecules, for example a standard "robustness set", a primary screening library (e.g. of otherwise unrelated compounds), a combinatorial library, a series of closely related chemical analogous and/or a set of known blockers and/or openers of other ion channels (such as a set of known blockers and/or openers of other (voltage gated) potassium channels); etc. Such sets or libraries will be clear to the skilled person, and may for instance include, but are not limited to, such commercially available chemical libraries such as the various libraries available from Tocris Cookson, Bristol, UK..

It will be clear to the skilled person that, for the screening of sets and/or libraries of compounds, it will often be preferred to carry out the method of the invention in a medium to high throughput fashion. For this, the methods of the invention are preferably carried out in an automated fashion (i.e. using suitable robotics) and/or in a suitable multi-well plate format, such as standard 6, 24, 48, 96, 384, 1536, or 3072 well-plates (in which each well of the multi-well plate may contain a separate sample of worms). Reference is *inter alia* made to the applications by Applicant referred to above - and in particular the International Application PCT/IB00/00575 (WO00/63427) - which not only describe medium-to-high throughput techniques for cultivating, handling and screening nematode worms in an automated, multi-well plate fashion; but also describe automated, non-visual techniques for measuring (changes in) phenotypical, physiological, behavioural and/or biochemical properties of nematodes (such as drinking, movement, defecation and egglaying), that may be used as a "significant biological change" in accordance with the present application.

In the methods of the invention, each individual sample of nematode worms will generally be exposed to a single compound to be tested, at a single concentration; with different samples being exposed either to two or more different compounds to be tested; to two different concentrations of the same compound (e.g. to establish a dose response curve for said compound), or a combination thereof.

It is also within the scope of the invention to expose the (sample of) nematodes to two or more compounds - at essentially the same time or sequentially (e.g. with an intermediate washing step) - for example to determine whether the two compounds have an effect which is the same or different from both the compounds separately (e.g. to provide a synergistic effect or an inhibitory or competitive effect). Furthermore, the methods of the invention may also involve the use of one or more reference samples, e.g. samples without any compound(s) present and/or with a predetermined amount of a known reference compound. It is also within the scope of the invention to use of two or more samples of nematode worms of different strains (such as two different strains expressing Kv4 channels and/or reference strains not expressing Kv4 channels as mentioned above), e.g. to compare the effect of the compound(s) to be tested on these different strains.

From the above, it will be clear to the skilled person that the method(s) described above will usually be embodied in an assay or a screen, and such assays and screens form further aspects of the invention.

For the purposes of the present disclosure, "agonists" of the Kv4 channel are generally defined as compounds which increase the flow of potassium ions through said Kv4 channel, whereas "antagonists" of the Kv4 channel are generally defined as compounds which decrease the flow of potassium ions through said Kv4 channel. In this respect, it should be noted that "blockers" of the Kv4 channel may act an agonist and/or an antagonist of the Kv4 channel (mainly depending on whether the compound affects the opening or the closing of the Kv4-channel) whereas "openers" of the Kv4 channel will usually act as agonists.

For example, agonists and antagonists may generally provide, and thus be identified by, the following non-limiting significant biological change(s) on hKv4-expressing strains:

**TABLE 3:**

| **Type of compound:** | **Nematode used:** | **Property determined:** | **Change observed:** |
|---|---|---|---|
| agonist | *pha-1:*hKv4.3; HD8:hKv4.3 | relaxation peak | increase |
| agonist | *pha-1:*hKv4.3; HD8:hKv4.3 | refractory period/ P-phase | decrease |
| agonist | *pha-1:*hKv4.3; HD8:hKv4.3 | pumping rate | decrease |
| agonist | *pha-1:*hKv4.3; HD8:hKv4.3 | drinking | decrease |
| antagonist | *pha-1*:hKv4.3; HD8:hKv4.3 | relaxation peak | decrease |
| antagonist | *pha-1*:hKv4.3; HD8:hKv4.3 | refractory period/ P phase | increase |
| antagonist | *pha-1*:hKv4.3; HD8:hKv4.3 | pumping rate | increase |
| agonist | *exp-2*:Kv4.3 | relaxation peak | increase |
| agonist | *exp-2*:hKv4.3 | refractory period/ P phase | decrease |
| weak agonist | *exp-2*:hKv4.3 | pumping rate | weak increase |
| strong agonist | *exp-*2:hKv4.3 | pumping rate | strong decrease |
| agonist | *exp-2*:hKv4.3 | drinking | decrease |
| antagonist | *exp*-2:hKv4.3 | relaxation peak | decrease (e.g. to level exp-2) |
| antagonist | *exp*-2:hKv4.3 | refractory period/ P phase | increase |
| antagonist | *exp-2:hKv4.3* | pumping rate | decrease |
| antagonist | *exp-2*:hKv4.3 | drinking | decrease |

By means of illustration only, using the methods of the invention, it has for example been found that 4-aminopyridine, a compound known to be a general antagonist of (voltage-gated) potassium channels, provides the following significant biological changes in the following hKv4.3 expressing nematodes (at the concentration and time of contact indicated):

**TABLE 4:**

| **Compound:** | **Nematode used:** | **Contact time/ concentration:** | **Change observed:** |
|---|---|---|---|
| 4-aminopyridine | *pha-1*;hKv4.3 | 500mM, 3hrs | increase in pharynx pumping rate |
| 4-aminopyridine | *exp*-2;hKv4.3 | 500mM, 3hrs | (small) decrease in pharynx pumping rate |
| 4-aminopyridine | *unc-119*;hKv4.3 | 500mM, 3hrs | increase in pharynx pumping rate |

In the pharmaceutical and/or veterinary field, the invention may be used to identify and develop compounds useful in the following disease area's (i.e. in preparing compositions for preventing, treating and/or curing the diseases/conditions indicated):
- heart diseases such as: arrythmia, tachycardia and congestive heart failure;
- diseases of the nervous systems such as: epilepsy; stroke, traumatic brain injury, anxiety, insomnia, Alzheimer's disease and Parkinson's syndrome.

Other disease area's in which the compounds may find use are hypertension and urinary incontinence

It is expected that for pharmaceutical use, openers of the Kv4 channel as may be identified/developed using the invention will be of particular importance.

For pharmaceutical use, the compounds may be used as a free acid or base, and/or in the form of a pharmaceutically acceptable acid-addition and/or base-addition salt (e.g. obtained with non-toxic organic or inorganic acid or base), in the form of a hydrate, solvate and/or complex, and/or in the form or a pre-drug, such as an ester. Such salts, hydrates, solvates, etc. and the preparation thereof will be clear to the skilled person; reference is for instance made to the salts, hydrates, solvates, etc. described in US-A-6,372,778, US-A-6,369,086 and US-6,369,067

Generally, for pharmaceutical use, the compounds may be formulated as a pharmaceutical preparation comprising at least one compound of the invention and at least one pharmaceutically acceptable carrier, diluent or excipient and/or adjuvant, and optionally one or more further pharmaceutically active compounds. By means of non-limiting examples, such a formulation may be in a form suitable for oral administration, for parenteral administration (such as by intravenous, intramuscular or subcutaneous injection or intravenous infusion), for topical administration, for administration by inhalation, by a skin patch, by an implant, by a suppository, etc.. Such suitable administration forms - which may be solid, semi-solid or liquid, depending on the manner of administration - as well as methods and carriers for use in the preparation thereof, will be clear to the skilled person; reference is again made to for instance US-A-6,372,778, US-A-3,696, 086 and US-6,369,067.

The pharmaceutical preparations are preferably in a unit dosage form, and may be suitably packaged, for example in a box, blister, vial, bottle, sachet, ampoule or in any other suitable holder or container (which may be properly labeled); optionally with one or more leaflets containing product information and/or instructions for use. Generally, such unit dosages will contain between 1 and 500 mg of the at least one compound of the invention, e.g. about 10, 25, 50, 100, 200, 500 or 1000 mg per unit dosage.

For pharmaceutical use, at least one compound will generally be administered in an amount of between 0.01 to 150 mg/kg body weight per day of the patient, divided over one or more daily doses. The amount(s) to be administered and the further treatment regimen may be determined by the treating clinician, depending on factors such as the age, gender and general condition of the patient and the nature and severity of the disease/symptoms to be treated.

In the agrochemical field, the invention may be used to identify compounds suitable for use in pesticides, insecticides, nematicides and/or other biocides or plant protection agents. For example, the compounds may be used to control the species listed in US-A-6,372,774. For this purpose, the compounds (or a suitable salt, hydrate or ester thereof) may be suitably formulated with one or more agrochemically acceptable carriers, to provide a formulation suitable for agrochemical use, as will be clear to the skilled person (reference is for example made to the formulations and uses described in US-A-6,372,774).

Also, the compounds and compositions may be used in the field of animal health, either to treat diseases (such as those mentioned above) in animals (e.g. mammals), and/or to control or prevent infestations of parasites or other harmful organisms in animals (e.g. mammals). Formulations of compounds of the invention suitable for these purposes will also be clear to the skilled person (reference is for example made to made to US-A-6,372,774).

The invention will now be further illustrated by means of the following non-limiting Experimental Part and by means of the non-limiting Figures, in which:
- Figures 1 shows an electropharyngeogram of a *C. elegans* strain UG 1547 expressing human Kv4.3 under the control of a myo-2 promoter (in an N2 background).
- Figures 2 and 3 are photographs showing the expression of the human Kv4.3 channel in the pharynx muscle of strain UG 1598 (Example III) and strain UG 1611 (Example IV), respectively. Photographs were taken following immunohistochemical staining with a rabbit anti-human Kv4.3 antibody, as described in Examples III and IV;
- Figure 4 is a graph showing the number of pharynx pumps per 30 seconds interval, with and without the presence of 4-aminopyridine, for strain UG 1598 (invention) and an N2 reference strain (comparative).
- Figure 5 is a graph showing the number of pharynx pumps per 30 seconds interval, with and without the presence of 4-aminopyridine, for strain UG 1611 (invention) and the *exp-2*(sa26as1426)V background (comparative);
- Figure 6 schematically shows expression vector pDW 2700 used in Example II.
- Figure 7 is a graph schematically showing the uptake of the fluorescent marker Calcein AM in the drinking assay of PCT/IB00/00575 by N2 (reference) and the strain UG 1598 (invention), in which the uptake of the fluorescent marker Calcein AM by N2 is taken at 100%.
- Figure 8 is a graph schematically showing the uptake of the fluorescent marker Calcein AM in the drinking assay of PCT/IB00/00575 by N2 and *exp-2* strains (reference) and the strain UG 1611 (invention), in which the uptake of the fluorescent marker Calcein AM by the *exp-2* background is taken at 100%.
- Figure 9 is a graph schematically showing the influence of 0.3 mM 4-aminopyridine on uptake of the fluorescent marker Calcein AM in the drinking assay of PCT/IB00/00575 by the *exp-2* strain (reference) and strain UG 1611 (invention), in which the uptake of the fluorescent marker Calcein AM by the *exp*-2 background without the presence of 4-aminopyridine is taken at 100%.

### EXPERIMENTAL PART

A full size cDNA encoding WT hKv4.3 was cloned from a cDNA library from human Brain. Unless indicated otherwise, all cloning steps were performed using standard protocols, i.e. as provided by the manufacturers of the reagents/kits used, and/or as described by Ausubel et al. and/or Sambrook et al., supra. A rabbit anti-human Kv4.3 antibody was P 0358, obtained from Sigma (St. Louis, Missouri). A anti-rabbit IgG Cy3 conjugate was C 2306, also obtained from Sigma (St. Louis, Missouri).

### Example I: Cloning of a full size cDNA encoding WT hKv4.3

PCR was performed in a 20µl reaction using Advantage®-GC 2 PCR (Clontech, Palo Alto, CA, U.S.A.) according to the manufacturers specifications, specific primer combination oGV 1/oGV4 and 1µl of marathon-Ready^{™} cDNA from human Brain (Clontech). The PCR conditions were as followed: An initial denaturation step at 95°C for 1', followed by 20 cycles of PCR (15" at 95°C, 3' at 68°C) and followed by 20 cycles of PCR (95°C for 15", at 68°C for 3' (+5"/cyclr)).

The resulting PCR products were analyzed by agarose gel electrophoresis and DNA of interest was isolated and cloned by TA-cloning into the pCR-XL-TOPO vector (Invitrogen). Upon the presence of appropriate restriction sites, a full length cDNA of the WT hKv4.3 cDNA was constructed (vide SEQ ID No. 4, with the corresponding amino acid sequence shown in SEQ ID no. 5).

The resulting plasmid was designated pGV7 (using primers combination oGV1 [SEQ ID No.1] /oGV4 [SEQ ID No.2])
oGV1: AGGGGTTTGCTGAACTAACTCCAAGCTGG
oGV4: TTCATTCCCCACTACCCACTCTGGCCCTCTGTCC

### Example II: Cloning of WT hKv4.3 cDNA in C. elegans Myo-2 expression vector

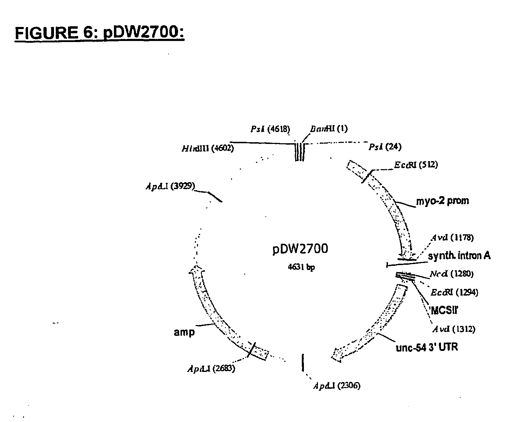

An 2172bp SpeI/XbaI fragment of pGV7 was isolated, purified and ligated into the NheI cloning site of the expression vector pDW2700 (schematically shown in Figure 6), thus bringing the hKv4.3 under the control of the myo-2 promoter present in said vector.

The resulting plasmid was designated pGV8

### Example III: transformation of a C. elegans pha-1 mutant with a human Kv4.3 encoding sequence.

*C. elegans* strain *pha-1*(e2123ts)III, obtained from the CGC (CGC strain GE24), was injected in the gonad with a mixture containing 5 ng/µl plasmid pGV8 (Example II), 20 ng/µl GFP-marker (pDW2821) and 5 ng/µl pBX rescue fragment for the *pha*-*1* phenotype, supplemented with genomic *C.elegans* DNA (Sau3A digest) to form a complex array (Kelly, Xu and Fire, Worm Breeders Gazette 14(1):64 (October 1, 1995)). Injection was performed according to the standard protocol described in I.A. Hope, supra.

The F 1 nematodes thus obtained were selected using the GFP marker for successful transformation with the pGV8 plasmid and expression of the hKv4.3 channel. The hKv4.3 expressing *pha-1* strain thus obtained was designated strain UG1598 and was deposited on April 25, 2002 with the BCCM under accession number LMBP 5851 CB.

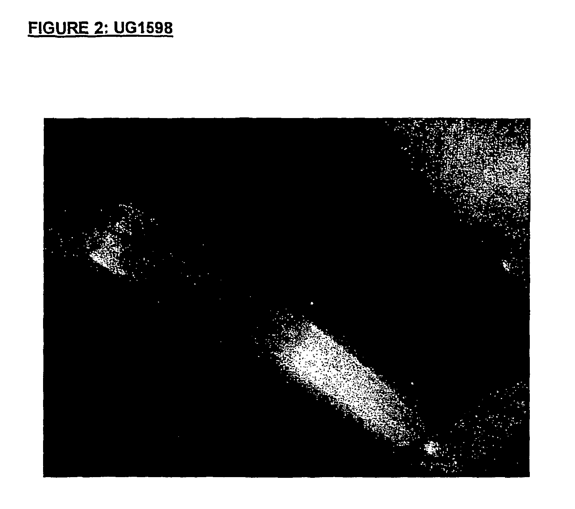

Expression of the hKv4.3 channel in the pharynx muscle was confirmed by fluorescent immunohistochemistry using the "whole mount technique" essentially as described in Steinbusch et al., Acta Histochem S35;85-106 (1998), using rabbit anti-human Kv4.3 antibody P 0358 (1/10 dilution) as the primary antibody and anti-rabbit IgG Cy3 conjugate C 2306 (1/1000 dilution) as the secondary antibody (vide Figure 2). From this, it appears that the expressed hKv4.3 is primarily, although not exclusively, located in the cell membrane, the expected location for a Kv4.3 like channel.

### Example IV: transformation of C. elegans exp-2 mutant with a human Kv 4.3 expressing sequence.

*C. elegans* strain *exp-2*(sa26ad1426)V, obtained from the CGC (CGC strain DA 1426), was injected in the gonad with a mixture containing 0,1 ng/µl plasmid pGV8 (Example II), 20 ng/µl GFP-marker (pDW2821), phenotype, supplemented with genomic *C.elegans* DNA (Sau3A digest) to form a complex array (Kelly, Xu and Fire, Worm Breeders Gazette 14(1):64 (October 1, 1995)). Injection was performed according to the standard protocol described in I.A. Hope, supra.

The F1 nematodes thus obtained were selected using the GFP marker for successful transformation with the pGV8 plasmid and expression of the hKv4.3 channel. The hKv4.3 expressing *exp-*2 strain thus obtained was designated strain UG1611 and was deposited on April 25, 2002 with the BCCM under accession number LMBP 5852CB.

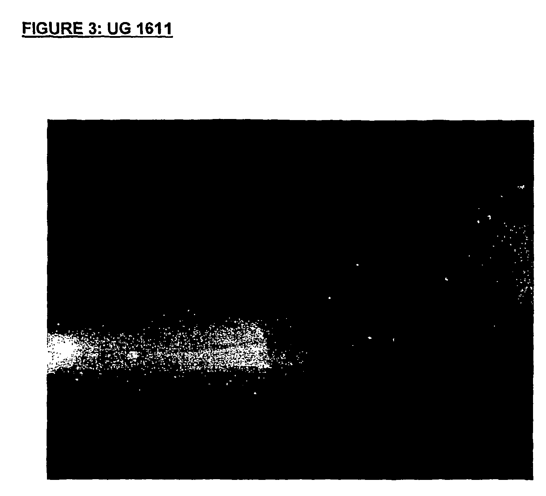

Expression of the hKv 4.3 channel in the pharynx muscle was confirmed by fluorescent immunohistochemistry using the "whole mount technique" essentially as described in Steinbusch et al., Acta Histochem S35;85-106 (1998), using rabbit anti-human Kv4.3 antibody P 0358 (1/10 dilution) as the primary antibody and anti-rabbit IgG Cy3 conjugate C 2306 (1/1000 dilution) as the secondary antibody (vide Figure 3).

### Example V: Influence of hKv4.3-expression on pharynx pumping of a C. elegans-pha-l strain.

The hKv4.3 expressing *C. elegans* strain UG 1598 (Example III) was investigated for the rate of pharynx pumping. As a comparison, an N2 background was used.

The nematodes were cultivated to L4 stage on an agar plate, and then kept overnight at 20° to reach the adult stage. The number of pharynx "pumps" (contractions) per 30 seconds were determined visually using a stopwatch.

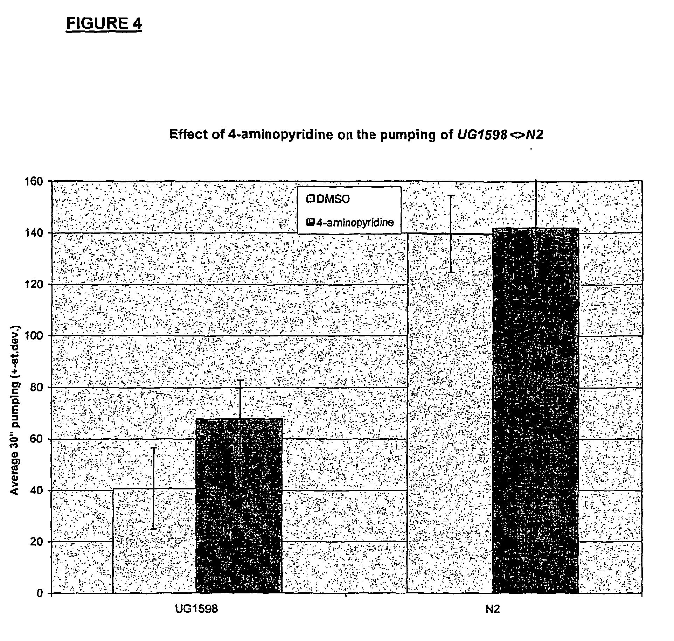

The results for are shown schematically in Figure 4. As can be seen, strain UG 1598 shows a severely reduced pharynx pumping rate compared to the background.

### Example VI: Influence of 4-aminopyridine on pharynx pumping in a hKv4.3 expressing pha-1 strain.

The influence of 4-aminopyridine, a known general antagonist of voltage-gated potassium channel, on the pharynx pumping rate of the hKv4.3 expressing *C. elegans* strain UG 1598 (Example III) was determined as follows.

The nematodes were cultivated to L4 stage on an agar plate, and then kept overnight at 20°C to reach the adult stage. The worms where then transferred to agar plates containing 0,5 mM 4-aminopyridine in DMSO. After 3 hours at 20°C, the number of pharynx "pumps" (contractions) per 30 seconds were determined visually using a stopwatch. As a comparison, the influence of 4-aminopyridine on an N2 background was also determined, in essentially the same manner.

The results for are shown schematically in Figure 4. As can be seen, 0,5 mM 4-aminopyridine increases the pharynx pumping rate of strain UG 1598, but has very little influence on the pharynx pumping rate of the background.

### Example VII: Influence of hKv4.3-expression on pharynx pumping of a C. elegans exp-2 strain.

The hKv4.3 expressing *C*. *elegans* strain UG 1611 (Example IV) was investigated for the rate of pharynx pumping. As a comparison, the original *exp-2* background was used.

The nematodes were cultivated to L4 stage on an agar plate, and then kept overnight at 20° to reach the adult stage. The number of pharynx "pumps" (contractions) per 30 seconds were determined visually using a stopwatch.

The results for are shown schematically in Figure 5. As can be seen, strain UG 1611 shows a slightly increased pharynx pumping rate (i.e. "rescue" of the exp-2 phenotype) compared to the *exp*-2 background.

### Example VIII: Influence of 4-aminopyridine on pharynx pumping in a hKv4.3 expressing exp-2 strain.

The influence of 4-aminopyridine, a known general antagonist of voltage-gated potassium channel, on the pharynx pumping rate of the hKv4.3 expressing *C. elegans* strain UG 1611 (Example IV) was determined as follows.

The nematodes were cultivated to L4 stage on an agar plate, and then kept overnight at 20°C to reach the adult stage. The worms where then transferred to agar plates containing 2 mM 4-aminopyridine in DMSO. After 3 hours at 20°C, the number of pharynx "pumps" (contractions) per 30 seconds were determined visually using a stopwatch. As a comparison, the influence of 4-aminopyridine on the original exp-2 background was also determined, in essentially the same manner.

The results for are shown schematically in Figure 5. As can be seen, 2 mM 4-aminopyridine decreases the pharynx pumping rate of strain UG 1611, but has very little influence on the pharynx pumping rate of the *exp-2* background.

### Example IX: Influence of hKv4.3-expression on drinking behaviour of a C. elegans pha-1 strain.

The hKv4.3 expressing *C. elegans* strain UG 1598 (Example III) was investigated for drinking behaviour by determining the uptake over time of the fluorescent dye Calcein AM, essentially as described in Examples 2 and 3 of the International application PCT/IB00/00575 by Applicant, using 96 well plates containing 100 nematodes/well. As a comparison, N2 was used.

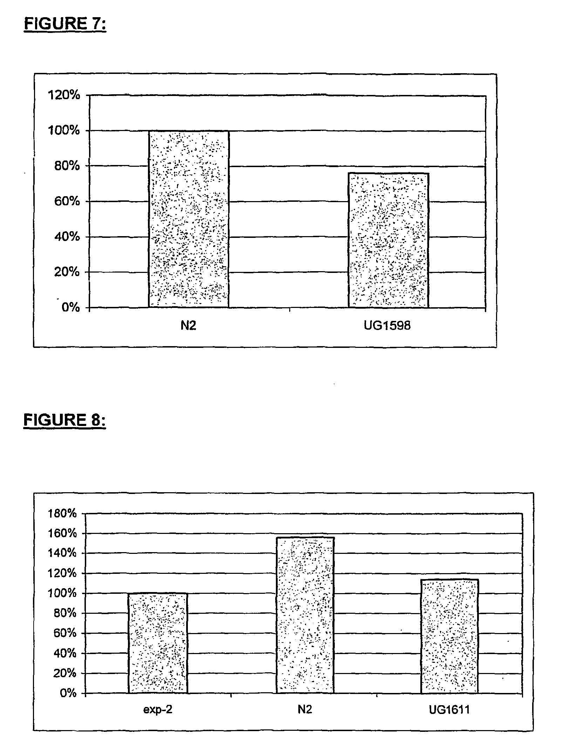

The results for are shown schematically in Figure 7, in which the uptake of Calcein AM by N2 strain is taken at 100%. As can be seen, the strain UG 1598 shows reduced drinking compared to N2 (i.e. 76% for UG 1598 compared to 100% for N2).

### Example X: Influence of hKv4.3-expression on drinking behaviour of a C. elegans exp-2 strain.

The hKv4.3 expressing *C. elegans* strain UG 1611 (Example IV) was investigated for drinking behaviour by determining the uptake over time of the fluorescent dye Calcein AM, essentially as described in Examples 2 and 3 of the International application PCT/IB00/00575 by Applicant, using 96 well plates containing 100 nematodes/well. As a comparison, the *exp*-2(sa26as1426)V background was used.

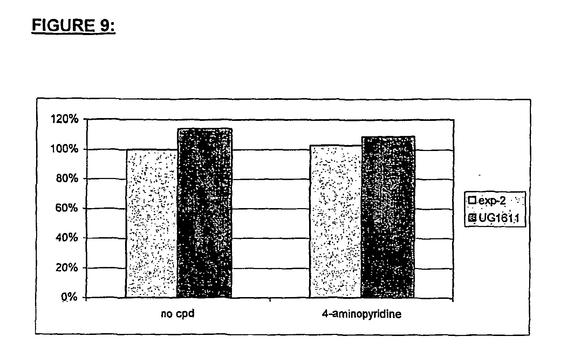

The results for are shown schematically in Figure 8, in which the uptake of Calcein AM by the *exp-2* strain is taken at 100%. As can be seen, the *exp-2* background shows reduced drinking compared to N2 (i.e. 100% for *exp-2* vs.156% for N2). In the strain UG 1611, expression of the hKv4.3 channel in the *exp-2* background increases the uptake of the marker to 114%.

### Example XI: Influence of 4-aminopyridine on drinking behaviour of a hKv4.3 expressing exp-2 strain.

The influence of mM 4-aminopyridine, a known general antagonist of voltage-gated potassium channel, on drinking behaviour of the hKv4.3 expressing *C. elegans* strain UG 1611 (Example IV) was determined by measuring the uptake of the fluorescent dye Calcein AM with and without the presence of 0.3 mM 4-aminopyridine, essentially as described in Examples 2 and 3 of the International application PCT/IB00/00575 by Applicant.

The results for are shown schematically in Figure 9, in which the uptake of Calcein AM by the *exp-2* strain without the presence of 4-aminopyridine is taken at 100%. As can be seen, in strain UG 1611, 0.3 mM 4-aminopyridine decreases the uptake of fluorescent marker in this drinking-assay from 114% to 109%; whereas for the *exp-2* background, 0.3 mM 4-aminopyridine increases the uptake of fluorescent marker in this drinking-assay from 100% to 103%.

### Example XII: Influence of expression of hKv4.3 on electropharyngeogram of C. elegans N2.

*C. elegans* strain N2 was injected in the gonad with a mixture containing 5 ng/µl plasmid pGV8 (Example II), 20 ng/µl GFP-marker (pDW2821) and 5 ng/µl pBX rescue fragment for the *pha-1* phenotype, supplemented with genomic *C.elegans* DNA (Sau3A digest) to form a complex array (Kelly, Xu and Fire, Worm Breeders Gazette 14(1):64 (October 1, 1995)). Injection was performed according to the standard protocol described in I.A. Hope, supra.

The F1 nematodes thus obtained were selected by using the GFP marker for successful transformation with the plasmid and expression of the hKv4.3 channel. The hKv4.3 expressing N2 strain was designated strain UG 1547.

An electropharyngeogram of the human Kv4.3 expressing strain UG 1547 was recorded using an Axopatch-1D amplifier (Axon instruments), essentially as described by Raizen and Avery, supra, and is shown in Figure 1. For comparison, reference is made to the EPG's for wild-type and *exp*-2 shown by Davis, Dent and Avery, Worm Breeders Gazette 14(4); 72 (1996), as well as the EPG's for wild-type shown in Raizen and Avery.

As can be seen from comparing the EPG of Figure 1 with the EPG's for wildtype from the above-cited references, expression of hKv4.3 in N2 leads to a significant reduction of the time between the E and R peaks (the "P phase") of the action potential ("AP") compared to wild-type, e.g. from a time in the range of 100-200 msec. for wild type to a time of less than 50 msec for the hKv4.3 expressing strain (e.g. about 10-20 msec or less). This easily and conveniently allows "Kv4.3-like" AP's to be distinguished from "wild-type like" or "normal" AP's in the recorded EPG.

Although the invention is not particularly limited to any specific explanation or hypothesis, it is assumed that this reduction of the P phase is mediated by the functionally expressed hKv4.3 channel, leading to a functional efflux of potassium ions. This is also confirmed by the following example.

### Example XIII: Influence of 4-aminopyridine on the electropharyngeogram of a hKv4.3 expressing C. elegans strain.

The influence of 4-aminopyridine on the EPG of a hKv4.3-expressing strain was studied using the hKv4.3-expressing *pha-1* strain UG 1598 of Example III. As mentioned in Example VI, exposing this strain UG 1598 to 4-aminopyridine leads to a reduction of pharynx pumping.

The EPG's for UG 1598 (using 4 pharynxes) were recorded essentially as described in Example XII. The EPG's of UG 1598 without 4-aminopyridine were recorded during a period of 5 minutes, upon which the pharynxes were exposed to 215mM 4-aminopyridine, and the EPG's were further recorded during a 5 minute period. The EPG's with and without the presence of 4-aminopyridine were then compared with each other and to the EPG for wild-type (see the references mentioned in Example XII).

As expected, the EPG's for UG1598 showed action potentials (AP's) with a significantly reduced P phase compared to wild-type, similar to the reduction of the P phase observed in the AP's for strain UG 1547 in Example XII. The number of "Kv4.3-like" AP's and the number of "normal" AP's during the 5 minute recording period were determined/counted from the recorded EPG's.

After exposure to the 4-aminopyridine, the number of "Kv4.3-like" AP's and the number of "normal" AP's during the 5 minute recording period were again determined/counted from the EPG recording.

Upon exposure to the 4-aminopyridine, a significant decrease of the relative number of "Kv4.3 like" AP's and a corresponding increase of the relative number of "normal" AP's was observed, compared to UG 1598 without 4-aminopyridine. In one experiment, the number of "normal" APs during the 5 minute recording period increased from 14 +/- 12 before adding 4-aminopyridine to 64 +/- 9 after adding 4-aminopyridine (results based on recording EPG's for 4 pharynxes; Paired Student's T-test analysis (using Excel software) gives a P-value of P<0.0161). These results *inter alia* confirm that the reduction of the P phase as seen in the Kv4.3 expressing strains is indeed mediated by the functionally expressed hKv4.3 proteins.

### Example XIV: Screening of a learning set of CNS-active compounds.

An integrated *C. elegans* strain expressing hKv4.3 under the control of a myo-2 promoter in a wild-type background was used to screen a set of 203 commercially available compounds with known CNS activity.

The readout used was drinking behaviour, measured as the uptake of Calcein AM over time (see Example IX). The screen was performed in an automated high throughout setting for measuring drinking behaviour at about 10,000 data points/screening day (For a general description of automated high throughput screening techniques for measuring drinking behaviour, reference is *inter alia* made to WO 00/63427 and WO 00/63425 by Applicant).

Out of the 203 compounds tested, eight compounds inhibited drinking by >60% inhibition of drinking and were confirmed as hits by means of dose response curves. This corresponds to a hit rate of 3.9%.

## Claims

1. Nematode worm, **characterized in that** said worm expresses a heterologous nucleotide sequence that encodes a functional voltage-gated potassium channel of the Kv4 family which is Kv4.2 or Kv4.3 or a mutant thereof having a degree of sequence identity, at the amino acid level, of at least 50% with the corresponding human Kv4.2 or Kv4.3 channel.

2. Nematode worm according to claim 1, **characterized in that** the nucleotide sequence encoding said voltage-gated potassium channel of the Kv4 family is represented by SEQ ID NO 3.

3. Nematode worm according to claim 1, **characterized in that** the nucleotide sequence encodes a mammalian Kv4 channel.

4. Nematode worm according to any of claims 1 to 3, **characterized in that** said voltage-gated potassium channel of the Kv4 family is expressed in at least the pharynx muscle, the body wall muscle and/or the vulva muscle.

5. Nematode worm according to claim 4, **characterized in that** said voltage-gated potassium channel of the Kv4 family is expressed in a mutant nematode that, on an electropharyngeogram, shows a reduced relaxation peak and/or refractory period, compared to wild type or N2.

6. Nematode worm according to any of claims 1-5, **characterized in that** said nematode, upon expression of said voltage-gated potassium channel of the Kv4 family, shows a change in muscle function, a change in muscle contraction and/or relaxation, a change in the rate of pharynx pumping and/or a change in drinking, compared to the nematode used to express said voltage-gated potassium channel of the Kv4 family.

7. Nematode worm according to claim 6 wherein the nematode, upon expression of said voltage-gated potassium channel of the Kv4 family, shows a change in function, contraction and/or relaxation of the pharynx muscle.

8. Nematode worm according to claim 7 wherein the change in function of the pharynx muscle is a change in polarization and/or repolarization, as seen on electropharyngeogram and/or intracellular electric recording.

9. Nematode worm according to any of claims 1, 3 or 4, **characterized in that** said nematode worm is C. elegans UG1598 (LMBP 5851CB).

10. Nematode worm according to any of claims 1-9, in which the voltage-gated potassium channel of the Kv4 family is co-expressed with one or more auxiliary proteins, acceptor molecules and/or subunits for voltage-gated potassium channels of the Kv4 family chosen from KChiPs, including but not limited to KChiP1, KChiP2, KChiP3, and Kvβ.

11. Nematode worm according to claim 10 wherein the voltage-gated potassium channel of the Kv4 family is co-expressed with one or more auxiliary proteins, acceptor molecules and/or subunits for KchiP 2.2.

12. Use of a nematode worm according to any of claims 1-11, in determining whether a compound interacts with the voltage gated potassium channel of the Kv4 family.

13. Method for identifying a compound that can interact with a voltage gated potasssium channel of the Kv4 family, said method comprising the steps of
(a) contacting a nematode worm according to any of claims 1-11 with said compound; and
(b) detecting and/or observing whether said compound causes a detectable change in or of said nematode worm;
wherein said detectable change identifies a compound that can interact with said Kv4 channel.

14. Method for identifying a compound that can be used in the prevention and/or treatment of a disease or disorder that is associated with and/or that is caused by a defect in a Kv4 channel, said method comprising the steps of:
(a) contacting a nematode worm according to any of claims 1-11 with said compound; and
(b) detecting and/or observing whether said compound causes a detectable change in or of said nematode worm;
wherein said detectable change identifies a compound that can be used in such prevention and/or treatment.

15. Method according to claim 14, in which the disease or disorder is a cardiac disorder that is associated with and/or that is caused by a defect in a Kv4 channel, and/or a disease or disorder of the nervous system that is associated with and/or that is caused by a defect in a Kv4 channel.

16. Method according to claim 15 wherein the cardiac disorder that is associated with and/or that is caused by a defect in a Kv4 channel is arrhythmia.

17. Method according to claim 15 wherein disease or disorder of the nervous system is a disease or disorder of the central nervous system.

18. Method according to any of claims 13-17, in which the at least one detectable change that is detected and/or observed in step (b) is a change in muscle function, a change in neuronal function, a change in the rate of pharynx pumping, a change in drinking, a change in movement, a change in egg laying and/or a change in defecation, compared to the nematode expressing said voltage-gated potassium channel of the Kv4 family prior to step (a).

## Patentansprüche

1. Fadenwurm (Nematode), **dadurch gekennzeichnet, dass** der Wurm eine heterologe Nukleotidsequenz exprimiert, welche einen funktionellen spannungsabhängigen (potentialabhängigen) Kaliumkanal der Kv4-Familie codiert, der Kv4.2 oder Kv4.3 oder eine Mutante davon mit einer Sequenzidentität von mindestens 50% auf der Aminosäureebene mit dem entsprechenden humanen Kv4.2 oder Kv4.3 Kanal darstellt.

2. Fadenwurm nach Anspruch 1, **dadurch gekennzeichnet, dass** die den spannungsabhängigen Kaliumkanal der Kv4-Familie codierende Nukleotidsequenz durch die SEQ ID NO 3 repräsentiert ist.

3. Fadenwurm nach Anspruch 2, **dadurch gekennzeichnet, dass** die Nukleotidsequenz einen Säugetier-Kv4-Kanal codiert.

4. Fadenwurm nach einem der Ansprüche 1 bis 3, **dadurch gekennzeichnet, dass** der spannungsabhängige Kaliumkanal der Kv4-Familie in zumindest dem Pharynxmuskel, dem Körperwandmuskel und/oder dem Vulvamuskel exprimiert ist.

5. Fadenwurm nach Anspruch 4, **dadurch gekennzeichnet, dass** der spannungsabhängige Kaliumkanal der Kv4-Familie in einem Mutanten-Fadenwurm exprimiert ist, welcher in einem Elektropharyngeogramm einen im Vergleich zum Wildtyp oder zu N2 verminderten Entspannungspeak und/oder eine verminderte Refraktärperiode zeigt.

6. Fadenwurm nach einem der Ansprüche 1 bis 5, **dadurch gekennzeichnet, dass** der Fadenwurm, nach Expression des spannungsabhängigen Kaliumkanals der Kv4-Familie, eine Änderung der Muskelfunktion, eine Änderung der Muskelkontraktion und/oder -relaxation, eine Änderung in der Rate des Pharynxpumpens und/oder eine Änderung beim Trinken aufweist, im Vergleich zu dem Fadenwurm, der zur Expression des spannungsabhängigen Kaliumkanals der Kv4-Familie verwendet wird.

7. Fadenwurm nach Anspruch 6, wobei der Fadenwurm nach Expression des spannungsabhängigen Kaliumkanals der Kv4-Familie eine Änderung der Funktion, Kontraktion und/oder Relaxation des Pharynxmuskels zeigt.

8. Fadenwurm nach Anspruch 7, wobei die Änderung in der Funktion des Pharynxmuskels eine Änderung der Polarisierung und/oder Repolarisierung ist, welche im Elektropharyngeogramm und/oder bei intrazellulärer elektrischer Aufzeichnung beobachtet wird.

9. Fadenwurm nach einem der Ansprüche 1, 3 oder 4, **dadurch gekennzeichnet, dass** das der Fadenwurm C. elegans UG1598 (LMBP 5851CB) ist.

10. Fadenwurm nach einem der Ansprüche 1 bis 9, bei welchem der spannungsabhängige Kaliumkanal der Kv4-Familie mit einem oder mehreren Hilfsproteinen, Akzeptormolekülen und/oder Untereinheiten für spannungsabhängige Kaliumkanäle der Kv4-Familie, ausgewählt aus KChiPs, einschließlich, aber nicht begrenzt auf, KChiP1, KChiP2, KChiP3 und Kvβ, coexprimiert ist.

11. Fadenwurm nach Anspruch 10, wobei der spannungsabhängige Kaliumkanal der Kv4-Familie mit einem oder mehreren Hilfsproteinen, Akzeptormolekülen und/oder Untereinheiten für KChiP 2.2 coexprimiert ist.

12. Verwendung eines Fadenwurms nach einem der Ansprüche 1 bis 11 zur Bestimmung, ob eine Verbindung mit dem spannungsabhängigen Kaliumkanal der Kv4-Familie interagiert.

13. Verfahren zur Identifizierung einer Verbindung, die mit einem spannungsabhängigen Kaliumkanal der Kv4-Familie interagieren kann, wobei das Verfahren die folgenden Schritte umfasst:
(a) in-Kontakt-bringen eines Fadenwurms nach einem der Ansprüche 1 bis 11 mit der Verbindung; und
(b) Detektieren und/oder Beobachten, ob die Verbindung eine detektierbare Änderung des Fadenwurms oder darin bewirkt;
wobei die detektierbare Änderung eine Verbindung identifiziert, die mit dem Kv4-Kanal interagieren kann.

14. Verfahren zur Identifizierung einer Verbindung, die bei der Prävention und/oder Behandlung einer Krankheit oder einer Störung verwendet werden kann, die mit einem Defekt in einem Kv4-Kanal assoziiert ist oder davon verursacht wird, wobei das Verfahren die folgenden Schritte umfasst:
(a) in-Kontakt-bringen eines Fadenwurms nach einem der Ansprüche 1 bis 11 mit der Verbindung; und
(b) Detektieren und/oder Beobachten, ob die Verbindung eine detektierbare Änderung des Fadenwurms oder darin bewirkt;
wobei die detektierbare Änderung eine Verbindung identifiziert, die bei der Prävention und/oder der Behandlung verwendet werden kann.

15. Verfahren nach Anspruch 14, wobei die Krankheit oder Störung eine Herzstörung ist, die mit einem Defekt in einem Kv4-Kanal assoziiert ist oder davon verursacht wird, und/oder eine Krankheit oder eine Störung des Nervensystems, die mit einem Defekt in einem Kv4-Kanal assoziiert ist oder davon verursacht wird.

16. Verfahren nach Anspruch 15, wobei die Herzstörung, die mit einem Defekt in einem Kv4-Kanal assoziiert ist oder davon verursacht wird, Arrhythmie ist.

17. Verfahren nach Anspruch 15, wobei die Krankheit oder Störung des Nervensystems eine Krankheit oder Störung des Zentralnervensystems ist.

18. Verfahren nach einem der Ansprüche 13 bis 17, wobei die mindestens eine Änderung, die in Schritt (b) detektiert und/oder beobachtet wird, eine Änderung der Muskelfunktion, eine Änderung der neuronalen Funktion, eine Änderung in der Rate des Pharynxpumpens, eine Änderung beim Trinken, eine Änderung der Bewegung, eine Änderung bei der Eiablage und/oder eine Änderung bei der Defäkation ist, im Vergleich zu dem Fadenwurm, der den spannungsabhängigen Kaliumkanal der Kv4-Familie vor dem Schritt (a) exprimiert.

## Revendications

1. Vers nématode, **caractérisé en ce que** ledit vers exprime une séquence nucléotidique hétérologue qui code un canal potassique sensible au voltage fonctionnel de la famille Kv4 qui est Kv4.2 ou Kv4.3 ou un de ses mutants, ayant un degré d'identité de séquence, sur le plan des acides aminés, d'au moins 50 % avec le canal humain Kv4.2 ou Kv4.3 correspondant.

2. Vers nématode selon la revendication 1, **caractérisé en ce que** la séquence nucléotidique codant ledit canal potassique sensible au voltage de la famille Kv4 est représentée par la SEQ ID N°3.

3. Vers nématode selon la revendication 1, **caractérisé en ce que** la séquence nucléotidique code un canal Kv4 d'un mammifère.

4. Vers nématode selon l'une quelconque des revendications 1 à 3, **caractérisé en ce que** ledit canal potassique sensible au voltage de la famille Kv4 est exprimé dans au moins le muscle du pharynx, le muscle d'une paroi du corps et/ou le muscle de la vulve.

5. Vers nématode selon la revendication 4, **caractérisé en ce que** ledit canal potassique sensible au voltage de la famille Kv4 est exprimé chez un nématode mutant, qui, au cours d'un électro-pharyngogramme, montre un pic de relâchement réduit et/ou une période réfractaire réduite, par rapport au type sauvage ou N2.

6. Vers nématode selon l'une quelconque des revendications 1 à 5, **caractérisé en ce que** ledit nématode, lors de l'expression dudit canal potassique sensible au voltage de la famille Kv4, montre un changement de fonction musculaire, un changement de contraction et/ou de relâchement musculaire, un changement de taux de pompage pharyngé et/ou un changement d'absorption de liquide, par rapport au nématode utilisé pour exprimer ledit canal potassique sensible au voltage de la famille Kv4.

7. Vers nématode selon la revendication 6, dans lequel le nématode, lors de l'expression dudit canal potassique sensible au voltage de la famille Kv4, montre un changement de fonction, de contraction et/ ou de relâchement du muscle du pharynx.

8. Vers nématode selon la revendication 7, dans lequel le changement de fonction du muscle pharyngé est un changement de polarisation et/ou repolarisation, comme on le voit sur l'électro-pharyngogramme et/ou l'enregistrement électrique intracellulaire.

9. Vers nématode selon l'une quelconque des revendications 1, 3 ou 4, **caractérisé en ce que** ledit vers nématode est *C. elegans* UG 1598 (LMBP 5851CB).

10. Vers nématode selon l'une quelconque des revendications 1 à 9, dans lequel ledit canal potassique sensible au voltage de la famille Kv4 est co-exprimé avec une ou plusieurs protéines auxiliaires, molécules acceptrices et/ou sous-unités pour des canaux potassiques sensible au voltage de la famille Kv4 choisies parmi les KChiP, y compris mais non limité à KChiP1, KChiP2, KChiP3 et Kvβ.

11. Vers nématode selon la revendication 10, dans lequel le canal potassique sensible au voltage de la famille Kv4 est co-exprimé avec une ou plusieurs protéines auxiliaires, molécules acceptrices et/ou sous-unités pour KchiP 2.2.

12. Utilisation d'un vers nématode selon l'une quelconque des revendications 1 à 11, pour déterminer si un composé interagit avec le canal potassique sensible au voltage de la famille Kv4.

13. Procédé pour identifier un composé qui peut interagir avec un canal potassique sensible au voltage de la famille Kv4, ledit procédé comprenant les étapes de :
(a) mettre en contact un vers nématode selon l'une quelconque des revendications 1 à 11 avec ledit composé ; et
(b) détecter et/ou observer si oui ou non ledit composé provoque un changement détectable dans ledit ou dudit vers nématode ;
dans lequel ledit changement détectable identifie un composé qui peut interagir avec ledit canal Kv4.

14. Procédé pour identifier un composé pouvant être utilisé dans la prévention et/ou le traitement d'une maladie ou d'un trouble associé et/ou provoqué par une anomalie dans un canal Kv4, ledit procédé comprenant les étapes de :
(a) mettre en contact un vers nématode selon l'une quelconque des revendications 1 à 11 avec ledit composé ; et
(b) détecter et/ou observer si oui ou non ledit composé provoque un changement détectable dans ledit ou dudit vers nématode ;
dans lequel ledit changement détectable identifie un composé qui peut être utilisé dans cette prévention et/ou ce traitement.

15. Procédé selon la revendication 14, dans-lequel la maladie ou le trouble est un trouble cardiaque associé et/ou provoqué par une anomalie dans un canal Kv4, et/ou une maladie ou un trouble du système nerveux associé et/ou provoqué par une anomalie dans un canal Kv4.

16. Procédé selon la revendication 15, dans lequel le trouble cardiaque associé et/ou provoqué par une anomalie dans un canal Kv4 est l'arythmie.

17. Procédé selon la revendication 15, dans lequel la maladie ou le trouble du système nerveux est une maladie ou un trouble du système nerveux central.

18. Procédé selon l'une quelconque des revendications 13 à 17, dans lequel le au moins un changement détectable qui est détecté et/ou observé à l'étape (b) est un changement de fonction musculaire, un changement de fonction neuronale, un changement du taux de pompage du pharynx, un changement dans l'absorption de liquide, un changement dans le déplacement, un changement dans le dépôt d'oeuf et/ou un changement de défécation, par rapport au nématode exprimant ledit canal potassique sensible au voltage de la famille Kv4 avant l'étape (a).


### SEQUENCE LISTING

<110> Devgen N.V.
<120> Methods for identifying and developing compounds that interact with voltage-gated potassium channels of the Kv4 family, optionally with auxiliary proteins such as KChiP proteins.
<130> P 02-004 PCT
<160> 5
<170> PatentIn version 3.1
<210> 1
   <211> 29
   <212> DNA
   <213> artificial sequence
<220>
   <223> primer oGV1
<400> 1
   aggggtttgc tgaactaact ccaagctgg 29
<210> 2
   <211> 34
   <212> DNA
   <213> artificial sequence
<220>
   <223> primer oGV4
<400> 2
   ttcattcccc actacccact ctggccctct gtcc 34
<210> 3
   <211> 1737
   <212> DNA
   <213> Caenorhabditis elegans
<400> 3
   atggcttcgg tggcggcgtg gctgccgttc gcgagagccg ccgctatcgg atgggttccc 60
   atttcgagac agcccatgcc acaggcccca gttgcaatac aggccaaaga cctcgcggtt 120
   gatcatgtct ctgacgaaaa actagcaatc aacatatcag gacgaaggtt cgaaacgtgg 180
   aagaacacgt tggaaaagtt cccagagact ctattggggt caaatgaaaa agagttcttt 240
   tatgatgaag acactgggga atactttttc gatagggatc cggacatatt ccgacacatt 300
   ctcacatttt atagaacggg aaaattgcat tatccccgtc atgaatgtct tgttgcatac 360
   gacgaagagc tcagtttctt tggaataatg cctgatttga tatcagattg ttgctatgaa 420
   gactataaag ataaaaaacg agaaaatcaa gaaagattac aagaagaacg tgtagaaaat 480
   gcggatatat caacattaaa attgtctctt aaagagaaaa tgtgggcggc attcgagaat 540
   ccgcacacaa catcaatagc acttgtattt tattatgtca ttggattttt tattgctgta 600
   tcagtgatgt gtaatatagt ggaaacaata ccttgtgggt atgaggataa tgtgtctgtg 660
   acgtgtggag aagcctatga agagcaattc tttgttatcg atacagcgtg tgttatcatt 720
   tttacgattg agtacttttt acggttgatt tctgcgccgg accgaatcaa atttatgagg 780
   tcaataatga gtgtaatcga tgtgattgcc ataatgcctt actacgttag tttggtacta 840
   actgacaata aggatgtatc cggcttgttt gtcactttaa gagttttccg tgtcttccga 900
   atattcaaat tctcacgaca ttcccaaggt cttcgaattc tgggttacac tcttaaatcg 960
   tgtgcaagtg aacttggatt ccttgtattt tcactggcaa tggcaattat catatttgcc 1020
   actattatgt attatgcgga gaaaaaggtc gacgcgacga gatttacctc gattccgtca 1080
   gctttctggt atacaattgt gactctaaca actcttggat acggagacat ggttccatca 1140
   acaataatgg gtaaaatagt tggaggagta tgctcactgt ctggagtgtt ggttattgct 1200
   cttccagttc cagttattgt cagcaatttc tctcgaattt atcatcaaaa tcagagagct 1260
   gacaagagaa aagcacagaa gaaagcccgt cttgctagaa ttcggatcgt taaaaacgcg 1320
   tctggactag cattattcaa taagaagaga gctcatgaag ctcggatgct agcatttgaa 1380
   cagggtcatc tctctttcga tgctctccga gacgaagata tcttcgaaat ccaacatcat 1440
   catcttcttc agtgtctaga aaaagcgacg gaacgtgaat ttgttgaatc agaagtgatg 1500
   tttgaaggtg gaagaaatac tcctccgccc agtgaaacag cttcattgaa aggaaaaaca 1560
   aagcgtaaaa ggcgattatg ctgtgtttca aaagaaaatg aagaaatgga agagttggat 1620
   cgagagactc gtgtaacatt caatcagaat atggatcaga tttgtgaatt accaaaacct 1680
   gatgaaatgg aactcaatca tcgaaataat gacaaaattt gcgtatcgca actgtaa 1737
<210> 4
   <211> 1968
   <212> DNA
   <213> human
<400> 4
   atggcggccg gagttgcggc ctggctgcct tttgcccggg ctgcggccat cgggtggatg     60
   ccggtggcca actgccccat gcccctggcc ccggccgaca agaacaagcg gcaggatgag    120
   ctgattgtcc tcaacgtgag tgggcggagg ttccagacct ggaggaccac gctggagcgc    180
   tacccggaca ccctgctggg cagcacggag aaggagttct tcttcaacga ggacaccaag    240
   gagtacttct tcgaccggga ccccgaagtg ttccgctgcg tgctcaactt ctaccgcacg    300
   gggaagctgc actacccgcg ctacgagtgc atctctgcct acgacgacga gctggccttc    360
   tacggcatcc tcccggagat catcggggac tgctgctacg aggagtacaa ggaccgcaag    420
   agggagaacg ccgagcggct catggacgac aacgactcgg agaacaacca ggagtccatg    480
   ccctcgctca gcttccgcca gaccatgtgg cgggccttcg agaaccccca caccagcacg    540
   ctggccctgg tcttctacta cgtgactggc ttcttcatcg ctgtctcggt catcaccaac    600
   gtggtggaga cggtgccgtg cggtacggtc ccgggcagca aggagctgcc gtgcggggag    660
   cgctactcgg tggccttctt ctgcctggac acggcgtgcg tcatgatctt caccgtggag    720
   tacctcctgc ggctcttcgc ggctcccagc cgctaccgct tcatccgcag cgtcatgagc    780
   atcatcgacg tggtggccat catgccctac tacatcggtc tggtcatgac caacaacgag    840
   gacgtgtccg gcgccttcgt cacgctccgg gtcttccgcg tcttcaggat cttcaagttt    900
   tcccgccact cccagggcct gcggatcctg ggctacacac tgaagagctg tgcctccgaa    960
   ctgggctttc ttctcttctc cctcaccatg gccatcatca tctttgccac tgtgatgttt   1020
   tatgccgaga agggctcctc ggccagcaag ttcacaagca tccctgcctc gttttggtac   1080
   accattgtca ccatgaccac actgggatac ggagacatgg tgcctaagac gattgcaggg   1140
   aagatcttcg gctccatctg ctccttgagt ggcgtcctgg tcattgccct gccagtccct   1200
   gtgattgttt ccaactttag ccggatttac caccagaatc agagagctga taaacgcagg   1260
   gcacaaaaga aggcccgcct tgccaggatc cgtgtggcca aaacaggcag ttcgaatgca   1320
   tacctgcaca gcaagcgcaa cgggctcctc aacgaggcgc tggagctgac gggcacccca   1380
   gaagaggagc acatgggcaa gaccacctca ctcatcgaga gccagcatca tcacctgctg   1440
   cactgcctgg aaaaaaccac tgggttgtcc tatcttgtgg atgatcccct gttatctgta   1500
   cgaacctcca ccatcaagaa ccacgagttt attgatgagc agatgtttga gcagaactgc   1560
   atggagagtt caatgcagaa ctacccatcc acaagaagtc cctcactgtc cagccaccca   1620
   ggcctcacta ccacctgctg ctcccgtcgt agtaagaaga ccacacacct gcccaattct   1680
   aacctgccag ctactcgcct gcgcagcatg caagagctca gcacgatcca catccagggc   1740
   agtgagcagc cctccctcac aaccagtcgc tccagcctta atttgaaagc agacgacgga   1800
   ctgagaccaa actgcaaaac atcccagatc accacagcca tcatcagcat ccccactccc   1860
   ccagcgctaa ccccagaggg ggaaagtcgg ccaccccctg ccagcccagg ccccaacacg   1920
   aacattcctt ccatagccag caatgttgtc aaggtctccg ccttgtaa                1968
<210> 5
   <211> 655
   <212> PRT
   <213> human
<400> 5